# EUROPEAN PATENT APPLICATION

(11) **EP 4 104 849 A1**
(43) Date of publication of application: **21.12.2022**
(21) Application number: 20914348.6
(22) Date of filing: 20.11.2020
(51) Int. Cl.: A61K 38/16, A61K 47/68, A61P 11/00, A61K 38/17, A61K 38/26, A61K 38/22, A61K 47/60, A61P 1/16

(54) **THERAPEUTIC USE OF LONG-ACTING CONJUGATE OF TRIPLE AGONIST HAVING ACTIVITY WITH RESPECT TO ALL OF GLUCAGON AND GLP-1 AND GIP RECEPTORS AGAINST LUNG DISEASE**

(30) Priority: 13.01.2020 KR 20200004379; 16.10.2020 KR 20200134344
(71) Applicant: Hanmi Pharm. Co., Ltd., Hwaseong-si, Gyeonggi-do 18536 (KR)
(72) Inventor: LEE, Seon Myeong, Hwaseong-si, Gyeonggi-do 18469 (KR); LEE, Jong Suk, Hwaseong-si, Gyeonggi-do 18469 (KR); KIM, Jung Kuk, Hwaseong-si, Gyeonggi-do 18469 (KR); LEE, Sang Hyun, Hwaseong-si, Gyeonggi-do 18469 (KR); OH, Euh Lim, Hwaseong-si, Gyeonggi-do 18469 (KR); JO, Hyo Sang, Hwaseong-si, Gyeonggi-do 18469 (KR); PARK, Eun Jin, Hwaseong-si, Gyeonggi-do 18469 (KR); LIM, Chong Yoon, Hwaseong-si, Gyeonggi-do 18469 (KR)
(74) Representative: Mewburn Ellis LLP
(86) International application number: PCT/KR2020/016535
(87) International publication number: WO 2021/145552

(57) **Abstract**

The present invention relates to preventive or therapeutic uses of a triple agonist having activities to all of glucagon, GLP-1, and GIP receptors, and/or conjugates thereof for lung disease.

## Description

### [Technical Field]

The present invention relates to preventive or therapeutic uses of a triple agonist having activities to all of glucagon, GLP-1, and GIP receptors, and/or conjugates thereof for lung disease.

### [Background Art]

The lungs are an organ mainly responsible for respiration, and lung diseases occur due to harmful substances, viruses, immune abnormalities, *etc.* Since lung diseases lead to decreased lung function and respiratory discomfort, *etc.,* appropriate treatment is required according to the cause of the disease.

Examples of lung-related diseases may include interstitial lung disease, progressive fibrosing interstitial lung disease, idiopathic interstitial pneumonias, non-specific interstitial pneumonia, pulmonary fibrosis, fibrosing interstitial lung diseases, idiopathic pulmonary fibrosis, alveolitis, pneumonia, emphysema, bronchitis, chronic obstructive pulmonary disease, combined pulmonary fibrosis and emphysema, asthma, or respiratory infectious disease (e.g., coronavirus disease (COVID-19)). Since these lung diseases are related to each other and thus, a combination of various symptoms appears, it is known that it is necessary to be careful in selecting a therapeutic agent. The main pathogenesis of lung disease includes lung injury, inflammatory response, and fibrosis.

Specifically, it is known that when an inflammatory reaction occurs in the lungs due to viruses, microorganisms, harmful substances, *etc.,* inflammatory cytokines (e.g., IL-1, IL-6, TNF-α) are secreted by alveolar macrophages, neutrophils are attracted, and proteases are secreted, *etc.,* and thus, the elasticity and structure of the lung tissue are damaged due to the secretion of proteases (e.g., elastase) that breaks down the fibers constituting the lung tissue, leading to fibrosis of the lungs. Inflammation and fibrosis of the lungs precede the progression to many lung diseases, and accordingly, these can be the fundamental mechanisms in the prevention and treatment of lung diseases.

Pulmonary fibrosis is a representative example of lung disease, and fibrosis is a disease in which excessive fibrous connective tissue is formed in an organ or tissue. Fibrosis refers to a state in which normal control is impossible during the wound healing process after tissue is damaged by various stresses (infection, chemical stimulation, radiation, *etc.)* in the human body. Fibrosis occurs in various organs such as the lungs, heart, liver, *etc.,* and is one of the fields with high unmet demand as no fundamental treatment has yet been developed.

Idiopathic pulmonary fibrosis (IPF), a pulmonary fibrosis of unknown cause, in which chronic fibrous interstitial pneumonia of unknown etiology progresses, is a disease in which fibrosis progresses due to continuous damage to alveolar epithelial cells, and treatment methods according to the administration of pirfenidone and nintedanib have been studied, but side effects such as decreased appetite, stomach weakness, side effects in the digestive systems, possible hepatotoxicity, photosensitivity rash, *etc.* have been reported.

In addition, chronic obstructive pulmonary disease (COPD), another representative example of lung disease, refers to a disease in which airway obstruction occurs gradually as the airway narrows due to an abnormal inflammatory response of the lungs caused by tobacco, air pollution, or toxic inhalants, which is broadly divided into chronic bronchitis and emphysema. In particular, smoking is known to be the main cause of chronic obstructive pulmonary disease. Smoking acts as a strong irritant in the lung tissue, increases the production of various pro-inflammatory factors, growth factors, oxidative substances and chemotactic factors, and activates the inflammatory signaling system, promoting the migration of many inflammatory cells, including neutrophils and macrophages to further exacerbate pulmonary inflammation. This eventually leads to abnormal changes in lung tissue, such as thickening of the airway wall and pulmonary fibrosis, thereby reducing lung function. Accordingly, improvement of lung inflammation is understood as one of the treatment methods for the prevention and treatment of chronic obstructive pulmonary disease.

Still another representative example of lung disease is lung damage caused by infection of the new coronavirus (2019-nCoV or SARS-CoV-2) and acute respiratory disease (COVID-19) caused thereby. The lungs are known as the main vulnerable organs because novel coronavirus, transmitted through the respiratory tract, penetrates into the cells through ACE2 and TMPRSS2, which are mainly expressed in type II alveolar epithelial cells. The main symptoms include fever, cough, *etc.,* and healthy adults are highly likely to recover over time. However, it is known that when the viral infection in the lungs is severe due to low initial immunity, it causes a serious inflammatory response according to lung damage and promotes fibrosis, which can be accompanied by symptoms such as acute respiratory distress syndrome (ARDS) and sepsis. Currently, drugs that suppress infection and proliferation of viruses or control lung inflammation are mainly used for COVID-19 treatment. Accordingly, improvement of lung inflammation and fibrosis is understood as one of the treatment methods for the prevention and treatment of COVID-19.

As such, since lung inflammation and fibrosis are the major causes of the onset and development of lung diseases, improvement of lung inflammation and fibrosis has been studied as a therapeutic mechanism for various lung diseases.

Meanwhile, glucagon-like peptide-1 (GLP-1) and glucose-dependent insulinotropic polypeptide (GIP) are representative gastrointestinal hormones and neuronal hormones, and are materials involved in the control of blood glucose levels according to food intake. Glucagon is a peptide hormone secreted by the pancreas and is involved in controlling the blood glucose levels along with the two materials described above. The development of therapeutic agents using drugs that can respectively or simultaneously act on GLP-1 receptors, GIP receptors, and glucagon receptors is being carried out (US 10,370,426, US 10,400,020).

Although various studies on lung diseases have been conducted so far, the development of practical and effective therapeutic agents is still insufficient, and accordingly, there is a need for continuous development of therapeutic agents.

### [Disclosure]

### [Technical Problem]

An object of the present invention is to provide a pharmaceutical composition for the prevention or treatment of lung disease, which includes a peptide having activities to a glucagon receptor, GLP-1 receptor, and GIP receptor, or a long-acting conjugate of the peptide.

Another object of the present invention is to provide a method for the prevention or treatment of lung disease, which includes administering a composition containing the peptide or a long-acting conjugate of the peptide to a subject in need thereof.

Still another object of the present invention is to provide a use of a composition containing the peptide or a long-acting conjugate of the peptide in the preparation of a medicament for the prevention or treatment of lung disease.

### [Technical Solution]

One aspect for implementing the present invention provides a pharmaceutical composition for the prevention or treatment of lung disease, which includes a peptide having activities to a glucagon receptor, a glucagon-like peptide-1 (GLP-1) receptor, and a glucose-dependent insulinotropic polypeptide (GIP) receptor.

In a specific embodiment, the pharmaceutical composition for the prevention or treatment of lung disease contains a pharmaceutically acceptable excipient; and a peptide including an amino acid sequence of any one of SEQ ID NOS: 1 to 102 in a pharmaceutically effective amount.

In a pharmaceutical composition according to any one of the previous specific embodiments, the peptide is in the form of a long-acting conjugate, and the long-acting conjugate is represented by the following Formula 1:

[Formula 1] X-L-F

wherein, in Formula 1 above,
X is a peptide of an amino acid sequence of any one of SEQ ID NOS: 1 to 102;
L is a linker including an ethylene glycol repeat unit;
F is an immunoglobulin Fc region; and
"-" represents a covalent bond between X and L and between L and F.

In a composition according to any one of the previous specific embodiments, the C-terminus of the peptide is amidated.

In a composition according to any one of the previous specific embodiments, the C-terminus of the peptide is amidated or has a free carboxyl group (-COOH).

In a composition according to any one of the previous specific embodiments, the peptide includes an amino acid sequence selected from the group consisting of SEQ ID NOS: 21, 22, 42, 43, 50, 64, 66, 67, 70, 71, 76, 77, 96, 97, and 100.

In a composition according to any one of the previous specific embodiments, the peptide includes an amino acid sequence selected from the group consisting of SEQ ID NOS: 21, 22, 42, 43, 50, 77, and 96.

In a composition according to any one of the previous specific embodiments, the amino acids at positions 16 and 20 from the N-terminus form a ring with each other in the peptide sequence.

In a composition according to any one of the previous specific embodiments, the formula weight of the ethylene glycol repeat unit portion in the L is in the range of 1 kDa to 100 kDa.

In a composition according to any one of the previous specific embodiments, the F is an IgG Fc region.

In a composition according to any one of the previous specific embodiments, the lung disease is interstitial lung disease (ILD), progressive fibrosing interstitial lung disease (PF-ILD), idiopathic interstitial pneumonias (IIP), non-specific interstitial pneumonia (NSIP), pulmonary fibrosis, fibrosing interstitial lung diseases (FILD), idiopathic pulmonary fibrosis (IPF), alveolitis, pneumonia, emphysema, bronchitis, chronic obstructive pulmonary disease, combined pulmonary fibrosis and emphysema (CPFE), asthma, or respiratory infectious disease.

In a composition according to any one of the previous specific embodiments, the respiratory infectious disease is an infectious disease caused by respiratory viruses, bacteria, mycoplasma, or fungi.

In a composition according to any one of the previous specific embodiments, the respiratory virus is any one selected from the group consisting of adenovirus, vaccinia virus, herpes simplex virus, parainfluenza virus, rhinovirus, varicella zoster virus, measle virus, respiratory syncytial virus, Dengue virus, HIV (human immunodeficiency virus), influenza virus, coronavirus, severe acute respiratory syndrome associated virus (SARS-associated virus), and middle east respiratory syndrome coronavirus (MERS-CoV).

In a composition according to any one of the previous specific embodiments, the coronavirus is SARS-CoV-2,

In a composition according to any one of the previous specific embodiments, the pharmaceutical composition has features of (i) inhibiting the activity of macrophages; and/or (ii) reducing the expression of IL-1β, IL-6, IL-12, or TNF-α when administered.

In a composition according to any one of the previous specific embodiments, the pharmaceutical composition has one or more following features when administered:
(i) inhibits myofibroblast differentiation;
(ii) reduces the expression of α-SMA, collagen 1α1, or fibronectin;
(iii) inhibits epithelial mesenchymal transition (EMT) of alveolar epithelial cells; and
(iv) reduces the expression of collagen 1α1 or collagen 1α3.

In a composition according to any one of the previous specific embodiments, the pharmaceutical composition is further administered with a mucolytic agent or a pharmaceutically acceptable salt thereof.

In a composition according to any one of the previous specific embodiments, the mucolytic agent is one or more selected from the group consisting of ambroxol, *N-*acetylcysteine, *N*-acetylin, carbocysteine, domiodol, fudosteine, bromhexine, erdosteine, letostine, lysozyme, mesna, sobrerol, stepronin, tiopronin, tyloxapol, carbocisteine, dornase alfa, eprazinone, letosteine, neltenexine, and mecysteine.

In a composition according to any one of the previous specific embodiments, the peptide and mucolytic agent or a pharmaceutically acceptable salt thereof are administered simultaneously, sequentially, or in reverse order.

In a composition according to any one of the previous specific embodiments, the lung disease is pneumonia (pulmonary inflammation) or pulmonary fibrosis caused by coronavirus disease-19 (COVID-19).

In a composition according to any one of the previous specific embodiments, the F is a dimer consisting of two polypeptide chains, and one end of L is linked to only one of the two polypeptide chains.

Another aspect for implementing the present invention provides a method for the prevention or treatment of lung disease, which includes administering the peptide or a composition containing the peptide to a subject in need thereof.

Still another aspect for implementing the present invention provides a use of the peptide or a composition containing the peptide in the preparation of a medicament for the prevention or treatment of lung disease.

Yet another aspect for implementing the present invention provides a use of the peptide or a composition containing the peptide in the prevention or treatment of lung disease.

### [Advantageous Effects]

The triple agonist or conjugate thereof according to the present invention has activities for a glucagon receptor, a glucagon-like peptide-1 (GLP-1) receptor, and a glucose-dependent insulinotropic polypeptide (GIP) receptor, and thus can exhibits an effect for preventing or treating lung disease.

### [Brief Description of Drawings]

FIG. 1 is a diagram confirming the change in the expression level of inflammatory cytokines in lung tissue according to the treatment of the long-acting conjugate of the triple agonist *in vivo.*
FIG. 2 is a diagram confirming the effect of improving emphysema according to the treatment of the long-acting conjugate of the triple agonist *in vivo.*
FIG. 3 is a diagram confirming the change in expression level of myofibroblast differentiation markers (a-SMA, collagen 1α1, and fibronectin) in lung fibroblasts (MRC5 cells) according to the treatment of the long-acting conjugate of the triple agonist.
FIG. 4 is a diagram confirming the change in the expression level of epithelial mesenchymal transition (EMT) markers (collagen 1α1 and collagen 1α3) in lung alveolar epithelial cells (A549 cells) according to the treatment of the long-acting conjugate of the triple agonist.
FIG. 5 is a diagram confirming the effect of improving fibrosis of lung tissue in BLM mice according to the treatment of the long-acting conjugate of the triple agonist *in vivo.*
FIG. 6 is a diagram confirming the change in the survival rate of BLM mice according to the treatment of the long-acting conjugate of the triple agonist.

### [DETAILED DESCRIPTION OF THE INVENTION]

Hereinafter, the present invention will be described in more detail.

Meanwhile, each of the explanations and exemplary embodiments disclosed herein can be applied to each other explanation and exemplary embodiment. That is, all of the combinations of various factors disclosed herein belong to the scope of the present invention. Moreover, the scope of the present invention should not be limited by the specific disclosure provided hereinbelow.

Throughout the entire specification of the present invention, not only the conventional one-letter and three-letter codes for naturally occurring amino acids, but also those three-letter codes generally allowed for other amino acids, such as α-aminoisobutyric acid (Aib), Sar (*N*-methylglycine), and α-methyl-glutamic acid, *etc.* are used. Additionally, the amino acids mentioned herein are abbreviated according to the nomenclature rules of IUPAC-IUB as follows:

| | |
|---|---|
| alanine (Ala, A) | arginine (Arg, R) |
| asparagine (Asn, N) | aspartic acid (Asp, D) |
| cysteine (Cys, C) | glutamic acid (Glu, E) |
| glutamine (Gln, Q) | glycine (Gly, G) |
| histidine (His, H) | isoleucine (Ile, I) |
| leucine (Leu, L) | lysine (Lys, K) |
| methionine (Met, M) | phenylalanine (Phe, F) |
| proline (Pro, P) | serine (Ser, S) |
| threonine (Thr, T) | tryptophan (Trp, W) |
| tyrosine (Tyr, Y) | valine (Val, V) |

One aspect for implementing the present invention provides a pharmaceutical composition for the prevention or treatment of lung disease, which includes a peptide having activities to a glucagon receptor, a glucagon-like peptide-1 (GLP-1) receptor, and a glucose-dependent insulinotropic polypeptide (GIP) receptor.

In one embodiment, the peptide may include an amino acid sequence of any one of SEQ ID NOS: 1 to 102.

In another embodiment, the pharmaceutical composition for the prevention or treatment of lung disease may be a pharmaceutical composition which includes a pharmaceutically acceptable excipient; and a peptide including an amino acid sequence of any one of SEQ ID NOS: 1 to 102 in a pharmaceutically effective amount.

In the present invention, the "peptide having activities to a glucagon receptor, a GLP-1 receptor, and a GIP receptor" can be used interchangeably with a "triple agonist".

Such a peptide includes various materials which have a significant level of activities to glucagon, GLP-1, and GIP receptors (e.g., various peptides).

Although not particularly limited, the triple agonist having a significant level of activities to glucagon, GLP-1, and GIP receptors may exhibit *in vitro* activities which are about 0.001% or higher, about 0.01% or higher, about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, and about 100% or higher, to one or more receptors, specifically two or more receptors, and more specifically all three of the receptors among the glucagon, GLP-1, and GIP receptors, compared to native ligands of the corresponding receptors (native glucagon, native GLP-1, and native GIP), and activity ranges with a significant increase are included without limitation.

In particular, the activities to receptors may include, for example, those cases where the *in vitro* activities are 0.1% or higher, 1% or higher, 2% or higher, 3% or higher, 4% or higher, 5% or higher, 6% or higher, 7% or higher, 8% or higher, 9% or higher, 10% or higher, 20% or higher, 30% or higher, 40% or higher, 50% or higher, 60% or higher, 70% or higher, 80% or higher, 90% or higher, 100% or higher, and about 200% or higher compared to native ligands, but the activities are not limited thereto.

As used herein, the term "about" refers to a range including all of ± 0.5, ± 0.4, ± 0.3, ± 0.2, ± 0.1, *etc.,* and it includes all of the values equivalent to those which come immediately after the term "about" or those in a similar range, but is not limited thereto.

The method for measuring the *in vitro* activity of the triple agonist may be referred to Experimental Example 1 of the present invention, but the method is not particularly limited thereto.

Meanwhile, the triple agonist is characterized by having one or more of the activities of i) to iii) described below, two or more of the activities, specifically all three activities, and particularly a significant activity thereof:
i) activation of a GLP-1 receptor; ii) activation of a glucagon receptor; and iii) activation of a GIP receptor.

In particular, the activation of receptors may include, for example, those cases where the *in vitro* activities are about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, and about 100% or higher, compared to native ligands, but the activities are not limited thereto.

Additionally, the peptide may be one which has an increased *in vivo* half-life compared to any one of native GLP-1, native glucagon, and native GIP, but the peptide is not particularly limited thereto.

Although not particularly limited, the peptide may be a peptide which is non-naturally occurring.

The peptide may be an analog of native glucagon, but is not particularly limited thereto. Specifically, the native glucagon analog includes peptides which have at least one difference in the amino acid sequence compared to that of native glucagon; peptides which are modified via modification of the native glucagon sequence; and mimetics of the native glucagon.

Meanwhile, native glucagon may have the following amino acid sequence, but is not particularly limited thereto:
His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr (SEQ ID NO: 118)

Specifically, the peptide may be an analog of native glucagon in which a variation selected from the group consisting of substitution, addition, deletion, modification, and a combination thereof has occurred on at least one amino acid of the native glucagon sequence, but the peptide is not particularly limited thereto.

Additionally, the substitution of an amino acid includes both a substitution with an amino acid and a substitution with a non-native compound.

Additionally, the addition may be performed at the N-terminus and/or C-terminus of a peptide. Meanwhile, the length of the amino acid to be added is not particularly limited, but 1 or more, 2 or more, 3 or more, 4 or more, 5 or more, 6 or more, 7 or more, 8 or more, 9 or more, 10 or more, and 11 or more amino acids may be added, and in a broad sense, the addition may include an addition of a polypeptide, but the addition is not particularly limited thereto.

More specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 amino acids selected from the group consisting of amino acids at positions 1, 2, 3, 7, 10, 12, 13, 14, 15, 16, 17, 18, 19, 20, 21, 23, 24, 27, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

Even more specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, or 19 amino acids selected from the group consisting of amino acids at positions 1, 2, 3, 10, 12, 13, 14, 15, 16, 17, 18, 19, 20, 21, 23, 24, 27, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

Even more specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, or 17 amino acids selected from the group consisting of amino acids at positions 1, 2, 3, 10, 13, 14, 15, 16, 17, 18, 19, 20, 21, 23, 24, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

Even more specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, or 14 amino acids selected from the group consisting of amino acids at positions 1, 2, 13, 16, 17, 18, 19, 20, 21, 23, 24, 27, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

The amino acids to be introduced may be selected from the group consisting of tyrosine, α-methyl-glutamic acid, Aib, methionine, glutamic acid, histidine, lysine, leucine, isoleucine, glutamine, valine, glycine, alanine, cysteine, serine, alanine, aspartic acid, and arginine, but the amino acids to be introduced are not particularly limited thereto.

For example, the amino acid sequence(s) to be added may be one or more amino acid sequences derived from a native GLP-1 amino acid sequence, a native GIP amino acid sequence, or a native exendin-4 amino acid sequence.

Such a peptide may include an intramolecular bridge (e.g., a covalent crosslinking or non-covalent crosslinking), and specifically, may be in the form including a ring, for example, may be in the form where a ring is formed between the 16^{th} amino acid and the 20^{th} amino acid of the peptide, but the peptide is not particularly limited thereto.

A non-limiting example of the ring may include a lactam bridge (or a lactam ring).

Additionally, the peptide includes all of those which are modified to include a ring, or include an amino acid capable of forming a ring in a target position.

For example, the peptide may be one where the amino acid pair at positions 16 and 20 is substituted with glutamic acid or lysine, which can form a ring, but the peptide is not limited thereto.

Such a ring may be formed between amino acid side chains within the peptide; for example, the ring may be in the form where a lactam ring is formed between a side chain of lysine and a side chain of glutamic acid, but the ring is not particularly limited thereto.

Examples of the peptide prepared by a combination of these methods may include peptides, in which the amino acid sequences thereof differ from that of native glucagon in at least one amino acid, and the α-carbon in the N-terminus of the amino acid residue is removed, while having activities to a glucagon receptor, a GLP-1 receptor, and a GIP receptor, *etc.,* but the peptide is not limited thereto, and the peptide applicable to the present invention may be prepared by a combination of various methods for the preparation of analogs.

Additionally, with respect to the peptide of the present invention, some of the amino acids may be substituted with other amino acids or non-natural compounds to avoid recognition by a degradation enzyme for increasing the *in vivo* half-life of the peptide, but the peptide is not particularly limited thereto.

Specifically, the peptide may be one in which the *in vivo* half-life is increased by avoiding recognition by the degradation enzyme through a substitution of the 2^{nd} amino acid sequence among the amino acid sequences of the peptide, but any substitution or modification of amino acids to avoid recognition by an *in vivo* degradation enzyme is included without limitation.

Additionally, such a modification for preparing a peptide includes all of the modifications using L-type or D-type amino acids and/or non-natural amino acids; and/or a modification of native sequence, for example, a modification of a side chain functional group, an intramolecular covalent bonding (e.g., ring formation between side chains), methylation, acylation, ubiquitination, phosphorylation, aminohexanation, biotinylation, *etc.*

Additionally, the modification also includes all of those where one or more amino acids are added to the amino and/or carboxy terminus of native glucagon.

As the amino acids for the substitution or addition, not only the 20 amino acids commonly found in human proteins, but also atypical or non-naturally occurring amino acids may be used. Commercial sources of atypical amino acids include Sigma-Aldrich, ChemPep Inc., and Genzyme Pharmaceuticals. The peptides, where these amino acids are included, and typical peptide sequences may be synthesized and purchased from commercial peptide synthesis companies (e.g., American Peptide Company, Bachem (USA), or Anygen (Korea)).

Amino acid derivatives may be obtained in the same manner, and as one such example, 4-imidazoacetic acid, *etc.* may be used.

Additionally, the peptide according to the present invention may be in the form of a variant where the N-terminus and/or C-terminus, *etc.* of the peptide is chemically modified or protected by organic groups, or amino acids may be added to the terminus of the peptide, for its protection from proteases *in vivo* while increasing its stability.

In particular, in the case of a chemically synthesized peptide, its N- and C-termini are electrically charged. Therefore, in order to remove such charge, the N-terminus of the peptide may be acetylated and/or the C-terminus of the peptide may be amidated, but the peptide modification is not particularly limited thereto.

Additionally, the peptide according to the present invention includes all of those in the form of the peptide itself, a salt thereof (e.g., a pharmaceutically acceptable salt thereof), or a solvate thereof. Additionally, the peptide may be in any pharmaceutically acceptable form.

The kind of the salt is not particularly limited. However, the salt is preferably one that is in a safe and effective form for a subject (e.g., a mammal), but the salt is not particularly limited thereto.

The term "pharmaceutically acceptable" refers to a material which can be effectively used for a desired use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic acids, organic acids, or bases. Examples of the suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* The salts derived from suitable bases may include alkali metals (e.g., sodium, potassium, *etc.*); alkali earth metals (e.g., magnesium); ammonium, *etc.*

As used herein, the term "solvate" refers to a complex formed between the peptide according to the present invention or salt thereof and a solvent molecule.

In one embodiment, the peptide may include an amino acid sequence represented by General Formula 1 below:
Xaa1-Xaa2-Xaa3-Gly-Thr-Phe-Xaa7-Ser-Asp-Xaa10-Ser-Xaa12-Xaa13-Xaa14-Xaa15-Xaa16-Xaa17-Xaa18-Xaa19-Xaa20-Xaa21-Phe-Xaa23-Xaa24-Trp-Leu-Xaa27-Xaa28-Xaa29-Xaa30-R1 (General Formula 1, SEQ ID NO: 103)

In General Formula 1 above,
Xaa1 is histidine, 4-imidazoacetyl, or tyrosine;
Xaa2 is glycine, α-methyl-glutamic acid, or Aib;
Xaa3 is glutamic acid or glutamine;
Xaa7 is threonine or isoleucine;
Xaa10 is leucine, tyrosine, lysine, cysteine, or valine;
Xaa12 is lysine, serine, or isoleucine;
Xaa13 is glutamine, tyrosine, alanine, or cysteine;
Xaa14 is leucine, methionine, or tyrosine;
Xaa15 is cysteine, aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is glutamic acid, glutamine, leucine, cysteine, or aspartic acid;
Xaa23 is isoleucine or valine;
Xaa24 is alanine, glutamine, cysteine, asparagine, aspartic acid, or glutamic acid;
Xaa27 is valine, leucine, or lysine;
Xaa28 is cysteine, lysine, alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, GKKNDWKHNIT (SEQ ID NO: 106), m-SSGAPPPS-n (SEQ ID NO: 107), or m-SSGQPPPS-n (SEQ ID NO: 108), or is absent, wherein:
   m is -Cys-, -Pro-, or -Gly-Pro-; and
   n is -Cys-, -Gly-, -Ser-, or -His-Gly-, or is absent.

Examples of the triple agonist may be those which include an amino acid sequence selected from the group consisting of SEQ ID NOS: 1 to 102; an amino acid sequence selected from the group consisting of SEQ ID NOS: 1 to 11 and SEQ ID NOS: 13 to 102; and those which (essentially) consist of an amino acid sequence selected from the group consisting of SEQ ID NOS: 1 to 11 and SEQ ID NOS: 13 to 102, but the triple agonist is not limited thereto.

In another embodiment, the triple agonist may be those which (essentially) consist of an amino acid sequence selected from the group consisting of SEQ ID NOS: 21, 22, 42, 43, 50, 64, 66, 67, 70, 71, 76, 77, 96, 97, and 100, but the triple agonist is not limited thereto.

In still another embodiment, the triple agonist may be those which (essentially) consist of an amino acid sequence selected from the group consisting of SEQ ID NOS: 21, 22, 42, 43, 50, 66, 67, 77, 96, 97 and 100, but the triple agonist is not limited thereto.

In yet another embodiment, the triple agonist may be those which (essentially) consist of an amino acid sequence selected from the group consisting of SEQ ID NOS: 21, 22, 42, 43, 50, 77 and 96, but the triple agonist is not limited thereto.

Additionally, although described as a peptide "consisting of" a particular SEQ ID NO: in the present invention, such description does not exclude a mutation that may occur by the addition of a meaningless sequence upstream or downstream of the amino acid sequence of the corresponding SEQ ID NO, or a mutation that may occur naturally, or a silent mutation thereof, as long as the peptide has an activity identical or corresponding to that of the peptide which consists of the amino acid sequence of the corresponding SEQ ID NO, and it obviously belongs to the scope of the present invention even when the peptide has such a sequence addition or mutation therein.

The above may be applicable to other specific embodiments or aspects of the present invention, but is not limited thereto.

Specifically, in General Formula 1 above, Xaa14 may be leucine or methionine, and Xaa15 may be cysteine, aspartic acid, or leucine.

Examples of such a peptide may include a peptide which includes an amino acid sequence selected from the group consisting of SEQ ID NOS: 1 to 11, 14 to 17, and 21 to 102; or a peptide which (essentially) consists of the same, but the peptide is not particularly limited thereto.

The peptide may significantly activate at least one of a glucagon receptor, a GLP-1 receptor, and a GIP receptor, but the peptide is not particularly limited thereto. Specifically, the peptide may be one which significantly activates a GLP-1 receptor, or additionally, significantly activates a glucagon receptor and/or a GIP receptor, but the peptide is not particularly limited thereto.

More specifically, the peptide may be one,
wherein in General Formula 1 above,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, glutamine, or cysteine;
Xaa14 is leucine, cysteine, or methionine;
Xaa15 is cysteine, leucine, glutamic acid, or aspartic acid;
Xaa17 is glutamine, arginine, isoleucine, cysteine, glutamic acid, or lysine;
Xaa18 is alanine, glutamine, arginine, or histidine;
Xaa19 is alanine, glutamine, valine, or cysteine;
Xaa20 is lysine, arginine, or glutamine;
Xaa21 is glutamic acid, glutamine, leucine, cysteine, or aspartic acid;
Xaa23 is isoleucine or valine;
Xaa24 is cysteine, alanine, glutamine, asparagine, glutamic acid, or aspartic acid; and
Xaa27 is leucine or lysine,
but the peptide is not particularly limited thereto.

More specifically, the peptide may be one,
wherein in General Formula 1 above,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, or cysteine;
Xaa14 is leucine or methionine;
Xaa15 is cysteine or aspartic acid;
Xaa17 is glutamine, arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid, cysteine, or aspartic acid;
Xaa23 is valine;
Xaa24 is alanine, glutamine, cysteine, asparagine, or aspartic acid; and
Xaa27 is leucine or lysine, but the peptide is not particularly limited thereto.

More specifically, the peptide may be one,
wherein in General Formula 1 above,
Xaa2 is α-methyl-glutamic acid or Aib;
Xaa7 is threonine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

Specifically, the peptide may be one,
wherein in General Formula 1 above,
Xaa1 is histidine or 4-imidazoacetyl;
Xaa2 is α-methyl-glutamic acid or Aib;
Xaa3 is glutamine;
Xaa7 is threonine;
Xaa10 is tyrosine;
Xaa12 is isoleucine;
Xaa13 is alanine or cysteine;
Xaa14 is methionine;
Xaa15 is aspartic acid;
Xaa16 is glutamic acid;
Xaa17 is isoleucine or lysine;
Xaa18 is alanine or histidine;
Xaa19 is glutamine or cysteine;
Xaa20 is lysine;
Xaa21 is aspartic acid;
Xaa23 is valine;
Xaa24 is asparagine;
Xaa27 is leucine;
Xaa28 is alanine or asparagine;
Xaa29 is glutamine or threonine; and
Xaa30 is cysteine or lysine, or is absent.

More specifically, the peptide may be one,
wherein in General Formula 1 above,
Xaa2 is glycine, α-methyl-glutamic acid, or Aib;
Xaa3 is glutamine;
Xaa7 is threonine;
Xaa10 is tyrosine, cysteine, or valine;
Xaa12 is lysine;
Xaa13 is tyrosine;
Xaa14 is leucine;
Xaa15 is aspartic acid;
Xaa16 is glycine, glutamic acid, or serine;
Xaa17 is glutamine, arginine, cysteine, or lysine;
Xaa18 is alanine, arginine, or histidine;
Xaa19 is alanine or glutamine;
Xaa20 is lysine or glutamine;
Xaa21 is glutamic acid, cysteine, or aspartic acid;
Xaa23 is valine;
Xaa24 is alanine, glutamine, or cysteine;
Xaa27 is leucine or lysine; and
Xaa29 is glycine, glutamine, threonine, or histidine, but the peptide is not particularly limited thereto.

Such a peptide may correspond to a case where the peptide has significant activation levels on both the GLP-1 receptor and glucagon receptor, or higher activation levels compared to that on the GIP receptor; a case where the peptide has significant activation levels on all of the GLP-1 receptor, glucagon receptor, and GIP receptor; or a case where the peptide has significant activation levels on both the GLP-1 receptor and GIP receptor and higher activation levels compared to that on the glucagon receptor; but the cases are not particularly limited thereto.

Examples of the peptide may include a peptide which includes an amino acid sequence selected from the group consisting of SEQ ID NOS: 8, 9, 21 to 37, 39, 42, 43, 49 to 61, 64 to 83, 85, 86, 88, 89, 91 to 93, and 95 to 102; or a peptide which (essentially) consists of the same, but the peptide is not particularly limited thereto.

In a specific embodiment, the peptide may include an amino acid sequence represented by General Formula 2 below.
Xaa1-Xaa2-Gln-Gly-Thr-Phe-Thr-Ser-Asp-Xaa10-Ser-Lys-Xaa13-Xaa14-Xaa15-Xaa16-Xaa17-Xaa18-Xaa19-Xaa20-Xaa21-Phe-Xaa23-Xaa24-Trp-Leu-Leu-Xaa28-Xaa29-Xaa30-Xaa31-Ser-Ser-Gly-Gln-Pro-Pro-Pro-Ser-Xaa40 (General Formula 2, SEQ ID NO: 104)

In General Formula 2 above, the peptide may be one where:
Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine or is absent.

More specifically, the peptide may be one, wherein in General Formula 2,
Xaa13 is alanine, tyrosine, or cysteine;
Xaa15 is aspartic acid or glutamic acid,
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine,
Xaa28 is cysteine, asparagine, or aspartic acid;
Xaa29 is glutamine, cysteine, or histidine; and
Xaa30 is cysteine, lysine, or histidine.

Examples of the peptide may include a peptide which includes an amino acid sequence selected from the group consisting of SEQ ID NOS: 21, 22, 42, 43, 50, 64 to 77, and 95 to 102; more specifically, a peptide which includes an amino acid sequence selected from the group consisting of SEQ ID NOS: 21, 22, 42, 43, 50, 64 to 77, and 96 to 102; or a peptide which (essentially) consists of the same, but the peptide is not particularly limited thereto.

In a specific embodiment, the peptide may include an amino acid sequence represented by General Formula 3 below.
Xaa1-Xaa2-Gln-Gly-Thr-Phe-Thr-Ser-Asp-Tyr-Ser-Lys-Xaa13-Leu-Asp-Glu-Xaa17-Xaa18-Xaa19-Lys-Xaa21-Phe-Val-Xaa24-Trp-Leu-Leu-Xaa28-Xaa29-Xaa30-Xaa31-Ser-Ser-Gly-Gln-Pro-Pro-Pro-Ser-Xaa40 (General Formula 3, SEQ ID NO: 105),

The peptide may be one, wherein in General Formula 3 above,
Xaa1 is histidine or tyrosine;
Xaa2 is α-methyl-glutamic acid or Aib;
Xaa13 is alanine, tyrosine or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine or is absent.

Examples of the peptide may include a peptide which includes an amino acid sequence selected from the group consisting of SEQ ID NOS: 21, 22, 42, 43, 50, 64 to 71, 75 to 77, and 96 to 102; or a peptide which (essentially) consists of the same, but the peptide is not particularly limited thereto.

Additionally, the peptide may be one, wherein in General Formula 1 above, R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent, but the peptide is not particularly limited thereto.

Additionally, the peptide of the present invention may be synthesized according to its length by a method well known in the art (e.g., by an automatic peptide synthesizer) and may be produced by genetic engineering technology.

Specifically, the peptide of the present invention may be prepared by a standard synthesis method, a recombinant expression system, or any other method known in the art. Accordingly, the peptide of the present invention may be synthesized by variety of methods including, for example, the methods described below:
(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture; or
(c) a method of performing an *in vitro* cell-free expression of a nucleic acid construct encoding a peptide and recovering the expression product therefrom; or
a method of obtaining fragments of a peptide by any combination of the methods (a), (b), and (c), followed by obtaining the peptide by linking the peptide fragments, and then recovering the peptide.

Additionally, the peptide having activities to a glucagon receptor, a GLP-1 receptor, and a GIP receptor may be in the form of a long-acting conjugate, in which a biocompatible material for increasing the *in vivo* half-life of a peptide is linked to a peptide having activities to a glucagon receptor, a GLP-1 receptor, and a GIP receptor. In the present specification, the biocompatible material can be used interchangeably with a carrier.

In the present invention, a conjugate of the peptide can exhibit an enhanced duration of efficacy compared to the peptide to which a carrier is not linked, and in the present invention, such a conjugate is referred to as a "long-acting conjugate".

Meanwhile, the conjugate may be one that is non-naturally occurring.

In one embodiment of the present invention, the long-acting conjugate may be one that is represented by Formula 1 below, but the long-acting conjugate is not limited thereto.

[Formula 1] X-L-F

wherein in Formula 1 above,
X is a peptide including an amino acid sequence of any one of SEQ ID NOS: 1 to 102;
L is a linker containing an ethylene glycol repeat unit;
F is an immunoglobulin Fc fragment or a derivative thereof; and
"-" represents a covalent bond between X and L and between L and F.

In the above conjugate, F is a material capable of increasing the half-life of X (i.e., a peptide having activities to a glucagon receptor, a GLP-1 receptor, and a GIP receptor; and specifically, a peptide including an amino acid sequence of any one of SEQ ID NOS: 1 to 102) and it corresponds to a constitution of the moiety that constitutes the conjugate of the present invention.

The F may be one which is linked to X by a covalent chemical bond or non-covalent chemical bond, and the F and the X may be linked to each other through L by a covalent chemical bond, non-covalent chemical bond or a combination thereof.

Specifically, the L may be a non-peptide linker, for example, a linker containing an ethylene glycol repeat unit.

In the present invention, the term "non-peptide linker" includes a biocompatible polymer in which two or more repeat units are linked. The repeat units are linked to each other through any covalent bond which is not a peptide bond. The non-peptide linker may be one constitution that constitutes the moieties of the conjugate of the present invention, and it corresponds to L in Formula 1 above.

As the non-peptide linker that can be used in the present invention, any polymer which has a resistance to proteases *in vivo* can be used without limitation. In the present invention, the non-peptide linker can be used interchangeably with a non-peptide polymer.

Although not particularly limited, the non-peptide linker may be a linker containing an ethylene glycol repeat unit (e.g., polyethylene glycol), and additionally, those derivatives which are already known in the art and the derivatives that can easily be prepared at the technological level of those skilled in the art are included in the scope of the present invention.

The repeat unit of the non-peptide linker may be an ethylene glycol repeat unit, and specifically, the non-peptide linker may be one which includes a functional group used for the preparation of the conjugate at an end while including an ethylene glycol repeat unit. The long-acting conjugate according to the present invention may be in the form in which X and F are linked through the functional group, but the long-acting conjugate is not limited thereto. In the present invention, the non-peptide linker may include two, or three or more functional groups, and each functional group may be the same as or different from each other, but the non-peptide linker is not limited thereto.

Specifically, the linker may be polyethylene glycol (PEG) represented by Formula 2 below, but the linker is not limited thereto: 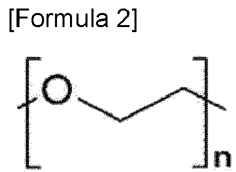 wherein n is 10 to 2,400, n is 10 to 480, or n is 50 to 250, but the range of n is not limited thereto.

In the long-acting conjugate above, the PEG moiety may include not only the -(CH₂CH₂O)ₙ- structure, but also an oxygen atom interposed between a linking element and the -(CH₂CH₂O)ₙ- structure, but the PEG moiety is not limited thereto.

Additionally, in a specific embodiment, the conjugate may have a structure in which an immunoglobulin fragment (F) is linked to a peptide (X) including the amino acid sequence of General Formula 1 by a covalent bond through a linker containing an ethylene glycol repeat unit, but the structure of the conjugate is not limited thereto.

The polyethylene glycol is a general term including all of the forms of homopolymers of ethylene glycol, PEG copolymers, and monomethyl-substituted PEG polymers (mPEG), but the polyethylene glycol is not particularly limited thereto.

The non-peptide linker to be used in the present invention may be any polymer which includes an ethylene glycol repeat unit having a resistance to proteases *in vivo* without limitation. The molecular weight of the non-peptide polymer may be in the range of exceeding 0 kDa to about 100 kDa, about 1 kDa to about 100 kDa, specifically about 1 kDa to about 20 kDa, or about 1 kDa to about 10 kDa, but the molecular weight of the non-peptide polymer is not limited thereto. Additionally, the non-peptide linker of the present invention, which is linked to the polypeptide corresponding to the F, may include not only a single kind of a polymer but also a combination of different kinds of polymers.

In a specific embodiment, both ends of the non-peptide linker can be linked to an amine group or thiol group of F (e.g., an immunoglobulin Fc region) and an amine group or thiol group of X, respectively.

Specifically, the non-peptide polymer may include a reactive group which can be linked to F (e.g., an immunoglobulin Fc region) and X at both ends thereof, respectively, and specifically, a reactive group which can be linked to an amine group located at the N-terminus or lysine, or a thiol group of cysteine of X or F (e.g., an immunoglobulin Fc region), but the non-peptide polymer is not limited thereto.

Additionally, the reactive group of the non-peptide polymer that can be linked to F (e.g., an immunoglobulin Fc region) and X may be selected from the group consisting of an aldehyde group, a maleimide group, and a succinimide derivative, but the reactive group is not limited thereto.

In the above, as an example of the aldehyde group, a propionaldehyde group or butyraldehyde group may be used, but the aldehyde group is not limited thereto.

In the above, as a succinimide derivative, succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate may be used, but the succinimide derivative is not limited thereto.

The non-peptide linker may be linked to X and F through these reactive groups, but the reactive groups are not particularly limited thereto.

Additionally, the final product produced through reductive amination by an aldehyde bond is much more stable than that linked by an amide bond. The aldehyde reactive group selectively reacts with a N-terminus at a low pH, while it can form a covalent bond with a lysine residue at a high pH (e.g., pH 9.0).

Additionally, the reactive groups at each end of the non-peptide linker may be the same as or different from each other. For example, the non-peptide linker may have a maleimide reactive group at one end, while having an aldehyde group, a propionaldehyde group, or a butyraldehyde group at the other end. However, the reactive groups are not particularly limited thereto as long as F (specifically, an immunoglobulin Fc region) can be linked to X at each end of the non-peptide linker.

For example, the non-peptide linker may include, as a reactive group, a maleimide group at one end, while including an aldehyde group, a propionaldehyde group, or a butyraldehyde group at the other end.

When a polyethylene glycol having a reactive hydroxy group at both ends thereof is used as the non-peptide polymer, the long-acting protein conjugate of the present invention may be prepared by activating the hydroxy group to various reactive groups by known chemical reactions or by using a commercially available polyethylene glycol having a modified reactive group.

In a specific embodiment, the non-peptide polymer may be one which is linked to a cysteine residue of X, and more specifically, to the -SH group of cysteine, but the non-peptide polymer is not limited thereto.

For example, the non-peptide polymer may be one which is linked to a peptide corresponding to the X, at a position of the 10^{th} cysteine residue, the 13^{th} cysteine residue, the 15^{th} cysteine residue, the 17^{th} cysteine residue, the 19^{th} cysteine residue, the 21^{st} cysteine residue, the 24^{th} cysteine residue, the 28^{th} cysteine residue, the 29^{th} cysteine residue, the 30^{th} cysteine residue, the 31^{st} cysteine residue, the 40^{th} cysteine residue, or the 41^{st} cysteine residue, but the non-peptide polymer is not particularly limited thereto.

Specifically, a reactive group of the non-peptide polymer can be linked to the -SH group of the cysteine residue, and all of the descriptions above can apply to the reactive group. In a case where a maleimide-PEG-aldehyde is used, the maleimide group is linked to the -SH group of X by a thioether bond, and the aldehyde group can be linked to F (specifically, a -NH₂ group of an immunoglobulin Fc) through a reductive amination reaction, but the linkage is not limited thereto, and this linkage is merely an embodiment.

In addition, in the conjugate above, the reactive group of the non-peptide polymer may be linked to -NH₂ located in the N-terminus of the immunoglobulin Fc region, but this linkage is merely an embodiment.

Meanwhile, the F may be an immunoglobulin Fc region, and more specifically, the immunoglobulin Fc region may be derived from IgG, but is not particularly limited thereto.

In the present invention, the term "immunoglobulin Fc region" refers to a region which includes the heavy chain constant region 2 (CH2) and/or heavy chain constant region 3 (CH3) portions, excluding the variable regions of immunoglobulin heavy and light chains. The immunoglobulin Fc fragment may be a constitution constituting the moiety of the conjugate of the present invention. The immunoglobulin Fc region may be used interchangeably with "immunoglobulin Fc fragment".

In the present invention, an Fc region includes not only the native sequence obtained by papain digestion of immunoglobulin, but also a derivative thereof (e.g., sequences in which one or more amino acid residues in the native sequence are modified by deletion, insertion, non-conservative or conservative substitution, or a combination thereof), and is thus different from that of the native form.

The F may have a structure in which two polypeptide chains are linked by a disulfide bond, or a structure in which two polypeptide chains are linked through a nitrogen atom in one of the two chains, but the structure of the F is not limited thereto. The linkage through the nitrogen atom may be linked to the epsilon amino atom or the N-terminus amino group of lysine via reductive amination.

The reductive amination reaction refers to a reaction in which an amine group or amino group of a reactant reacts with an aldehyde of another reactant (i.e., a functional group capable of reductive amination) to produce an amine, and an amine bond is formed by a reduction reaction thereafter. The reductive amination reaction is a reaction of organic synthesis widely known in the art.

In an embodiment, the F may be one which is linked through a nitrogen atom of the N-terminus proline, but the F is not limited thereto.

The immunoglobulin Fc region is one constitution constituting a moiety of the conjugate of Formula 1 of the present invention, and specifically, may correspond to F in Formula 1 above.

Such an immunoglobulin Fc region may include a hinge region in the heavy chain constant region, but is not limited thereto

In the present invention, the immunoglobulin Fc region may include a specific hinge sequence at the N-terminus.

As used herein, the term "hinge sequence" refers to a region which is located in the heavy chain and forms a dimer of immunoglobulin Fc regions through an inter-disulfide bond.

In the present invention, the hinge sequence may be a modified sequence in which part of the hinge sequence having the following amino acid sequence is deleted and thus there is only one cysteine residue in the sequence, but the hinge sequence is not limited thereto:
Glu-Ser-Lys-Tyr-Gly-Pro-Pro-Cys-Pro-Ser-Cys-Pro (SEQ ID NO: 119).

The hinge sequence may be one in which the 8^{th} or 11^{th} cysteine residue in the hinge sequence of SEQ ID NO: 119 is deleted and thus only one cysteine residue is included in the sequence. The hinge sequence of the present invention may consist of 3 to 12 amino acids, including only one cysteine residue, but the hinge sequence is not limited thereto. More specifically, the hinge sequence of the present invention may have the following sequences:
Glu-Ser-Lys-Tyr-Gly-Pro-Pro-Pro-Ser-Cys-Pro (SEQ ID NO: 120), Glu-Ser-Lys-Tyr-Gly-Pro-Pro-Cys-Pro-Ser-Pro (SEQ ID NO: 121), Glu-Ser-Lys-Tyr-Gly-Pro-Pro-Cys-Pro-Ser (SEQ ID NO: 122), Glu-Ser-Lys-Tyr-Gly-Pro-Pro-Cys-Pro-Pro (SEQ ID NO: 123), Lys-Tyr-Gly-Pro-Pro-Cys-Pro-Ser (SEQ ID NO: 124), Glu-Ser-Lys-Tyr-Gly-Pro-Pro-Cys (SEQ ID NO: 125), Glu-Lys-Tyr-Gly-Pro-Pro-Cys (SEQ ID NO: 126), Glu-Ser-Pro-Ser-Cys-Pro (SEQ ID NO: 127), Glu-Pro-Ser-Cys-Pro (SEQ ID NO: 128), Pro-Ser-Cys-Pro (SEQ ID NO: 129), Glu-Ser-Lys-Tyr-Gly-Pro-Pro-Ser-Cys-Pro (SEQ ID NO: 130), Lys-Tyr-Gly-Pro-Pro-Pro-Ser-Cys-Pro (SEQ ID NO: 131), Glu-Ser-Lys-Tyr-Gly-Pro-Ser-Cys-Pro (SEQ ID NO: 132), Glu-Ser-Lys-Tyr-Gly-Pro-Pro-Cys (SEQ ID NO: 133), Lys-Tyr-Gly-Pro-Pro-Cys-Pro (SEQ ID NO: 134), Glu-Ser-Lys-Pro-Ser-Cys-Pro (SEQ ID NO: 135), Glu-Ser-Pro-Ser-Cys-Pro (SEQ ID NO: 136), Glu-Pro-Ser-Cys (SEQ ID NO: 137), Ser-Cys-Pro (SEQ ID NO: 138).

More specifically, the hinge sequence may be one which includes the amino acid sequence of SEQ ID NO: 129 (Pro-Ser-Cys-Pro) or SEQ ID NO: 138 (Ser-Cys-Pro), but the hinge sequence is not limited thereto.

The immunoglobulin Fc region of the present invention may be in the form in which two molecules of the immunoglobulin Fc chain form a dimer due to the presence of a hinge sequence therein, and in addition, the conjugate of Formula 1 of the present invention may be in the form in which one end of the linker is linked to one chain of the dimeric immunoglobulin Fc regions, but the immunoglobulin Fc region and the conjugate of Formula 1 are not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end of the amino terminus or the terminal end. The immunoglobulin Fc region of the present invention may include a hinge sequence at the N-terminus, but the immunoglobulin Fc region is not limited thereto.

In addition, the immunoglobulin Fc region of the present invention may be an extended Fc region, which includes all or part of the heavy chain constant region 1 (CH1) and/or light chain constant region 1 (CL1) excluding the heavy chain and light chain variable regions of an immunoglobulin, as long as it has substantially the same or an improved effect compared to its native type. In addition, the immunoglobulin Fc region of the present invention may be a region in which some fairly long amino acid sequences corresponding to CH2 and/or CH3 are removed.

For example, the immunoglobulin Fc fragment of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain, and a CH4 domain and an immunoglobulin hinge region (or part of the hinge region); and 6) a dimer of each domain of the heavy chain constant region and a light chain constant region, but the immunoglobulin Fc region is not limited thereto.

In addition, in one embodiment of the long-acting conjugate of the present invention, the immunoglobulin Fc region F is a dimer consisting of two polypeptide chains, in particular, the Fc region dimers F and X are covalently linked through one and the same linker L containing an ethylene glycol repeat unit. In one specific embodiment, X is covalently linked to only one of the two polypeptide chains of the Fc region dimer F via a linker L. In a more specific example, only one molecule of X is covalently linked via L to one of the two polypeptide chains of the Fc region dimer F to which X is linked. In the most specific example, F is a homodimer.

In another embodiment of the long-acting conjugate of the present invention, it is also possible that two molecules of X are symmetrically bound to one Fc region in a dimeric form. In particular, the immunoglobulin Fc and X may be linked to each other by a non-peptide linker, but the linkage between the immunoglobulin Fc region and X is not limited to the embodiments described above.

In addition, the immunoglobulin Fc fragment of the present invention includes natural amino acid sequences as well as sequence derivatives thereof. The amino acid sequence derivative means that the sequence of the amino acid is different from that of its natural amino acid due to deletion, addition, non-conservative or conservative substitution, or a combination thereof in one or more amino acid residues in the sequence of the natural amino acid.

For example, amino acid residues at positions 214 to 238, 297 to 299, 318 to 322, or 327 to 331 in IgG Fc, which are known to be important for linkage, may be used as the sites suitable for modification.

Additionally, various types of derivatives are possible, for example, one where the site capable of forming a disulfide bond is removed; one where several N-terminal amino acids from native Fc are removed; one where a methionine residue is added to the N-terminus of native Fc, *etc.* Additionally, complement binding sites (e.g., C1q binding sites) or antibody-dependent cell-mediated cytotoxicity (ADCC) sites may be removed to remove the effector function. The techniques for preparing the sequence derivatives of these immunoglobulin Fc regions are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, *etc.*

The Fc derivatives described above may be those which exhibit the same biological activity as the Fc region of the present invention and have increased structural stability of the Fc region against heat, pH, *etc.*

Additionally, such an Fc region may be obtained from a native type isolated from humans or animals (e.g., cows, goats, pigs, mice, rabbits, hamsters, rats, guinea pigs, *etc.)* or may be recombinants or derivatives thereof obtained from transformed animal cells or microorganisms. In particular, the Fc region may be obtained from a native immunoglobulin by isolating a whole immunoglobulin from a living human or animal body and treating the isolated immunoglobulin with protease. When the whole immunoglobulin is treated with papain, it is cleaved into Fab and Fc regions, whereas when treated with pepsin, it is cleaved into pF'c and F(ab)₂ fragments. Fc or pF'c can be isolated using size exclusion chromatography, *etc.* In a more specific embodiment, the Fc region may be a recombinant immunoglobulin Fc region where a human-derived Fc region is obtained from a microorganism.

Additionally, the immunoglobulin Fc region may be in the form of native glycans, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc region where the glycans are removed from the Fc shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, an immunoglobulin Fc region in a deglycosylated or aglycosylated form may be more suitable to meet the original object of the present invention as a drug carrier.

As used herein, the term "deglycosylation" refers to an Fc region in which glycans are removed with an enzyme, and the term "aglycosylation" refers to an unglycosylated Fc region produced in prokaryotes, more specifically, *E. coli.*

Meanwhile, the immunoglobulin Fc fragment may be derived from humans or animals (e.g., cows, goats, pigs, mice, rabbits, hamsters, rats, guinea pigs, etc.), and in a more specific embodiment, it may be derived from humans.

Additionally, the immunoglobulin Fc region may be derived from IgG, IgA, IgD, IgE, IgM, or a combination or hybrid thereof. In a more specific embodiment, the immunoglobulin Fc region may be derived from IgG or IgM, which are among the most abundant proteins in human blood, and in an even more specific embodiment, it may be derived from IgG, which is known to enhance the half-lives of ligand-binding proteins. In an even yet more specific embodiment, the immunoglobulin Fc region may be an IgG4 Fc region, and in the most specific embodiment, it may be an aglycosylated Fc region derived from a human IgG4, but the immunoglobulin Fc region is not limited thereto.

Additionally, in a specific embodiment, the immunoglobulin Fc fragment, being a human IgG4 Fc fragment, may be in the form of a homodimer in which two monomers are linked through an inter-disulfide bond (an inter-chain form) between cysteines, which are the 3^{rd} amino acid of each monomer. In particular, each monomer of the homodimer independently has/or can have an internal disulfide bond between the cysteines at positions 35 and 95; and an internal disulfide bond between the cysteines at positions 141 and 199 (i.e., two internal disulfide bonds (an intra-chain form)). With respect to the number of amino acids, each monomer may consist of 221 amino acids, and the amino acids forming the homodimer may consist of a total of 442 amino acids, but the number of amino acids is not limited thereto. Specifically, the immunoglobulin Fc fragment may be one in which two monomers having the amino acid sequence of SEQ ID NO: 139 (consisting of 221 amino acids) form a homodimer through an inter-disulfide bond between cysteines, which are the 3^{rd} amino acid of each monomer, and in which the monomers of the homodimer independently form an internal disulfide bond between the cysteines at positions 35 and 95 and an internal disulfide bond between the cysteines at positions 141 and 199, but the immunoglobulin Fc fragment is not limited thereto.

Meanwhile, as used herein, the term "combination" means that polypeptides encoding single-chain immunoglobulin Fc regions of the same origin are linked to a single-chain polypeptide of a different origin to form a dimer or multimer. That is, it is possible to prepare a dimer or multimer from two or more fragments selected from the group consisting of Fc fragments of IgG Fc, IgA Fc, IgM Fc, IgD Fc, and IgE Fc.

As used herein, the term "hybrid" means that sequences corresponding two or more immunoglobulin Fc fragments of different origins are present in a single chain of an immunoglobulin constant region. In the present invention, various hybrid forms are possible. For example, the hybrid domain may be composed of one to four domains selected from the group consisting of CH1, CH2, CH3, and CH4 of IgG Fc, IgM Fc, IgA Fc, IgE Fc, and IgD Fc, and may further include a hinge region.

Meanwhile, IgG may also be divided into the IgG1, IgG2, IgG3, and IgG4 subclasses, and the present invention may include combinations or hybrids thereof. Preferred are the IgG2 and IgG4 subclasses, and most preferred is the Fc fragment of IgG4, which rarely has effector functions such as complement-dependent cytotoxicity (CDC).

In addition, the above-described conjugate may have an enhanced duration of efficacy compared to native GLP-1, GIP, or glucagon or to X which is not modified with F, and such a conjugate is not only in the above-described form, but also in the form encapsulated in biodegradable nanoparticles, but is not limited thereto.

A composition containing the peptide (e.g., the peptide itself or a long-acting conjugate form in which a biocompatible material is bound to the peptide) may be used for the prevention or treatment of lung disease.

As used herein, the term "prevention" refers to all activities that inhibit or delay the occurrence of lung disease by administering the above peptide (e.g., the peptide itself or a long-acting conjugate form in which a biocompatible material is bound to the peptide) or a composition containing the peptide, and the term "treatment" refers to all activities that improve or advantageously change the symptoms of lung disease by administering the above peptide (e.g., the peptide itself or a long-acting conjugate form in which a biocompatible material is bound to the peptide) or a composition containing the peptide.

As used herein, the term "administration" refers to the introduction of a particular material into a patient by any appropriate method, and the administration route of the composition is not particularly limited, but may be any conventional route that enables delivery of the composition to the target in the body (e.g., intraperitoneal administration, intravenous administration, intramuscular administration, subcutaneous administration, intradermal administration, oral administration, topical administration, intranasal administration, intrapulmonary administration, intrarectal administration, *etc.).*

The use of the triple agonist having activities to all of glucagon, GLP-1 and GIP receptors or a long-acting conjugate thereof remarkably increases the blood half-life and *in vivo* duration of efficacy, thus, there is a great advantage that the administration frequency is reduced to improve quality of life in chronic patients that suffer from daily injections, and accordingly, it is very useful for the treatment of lung disease. Further, the triple agonist or a long-acting conjugate thereof has effects of preventing lung disease, such as delaying the recurrence period of symptoms of lung disease, and/or significantly reducing symptoms of lung disease, and thus is very useful for the prevention and/or treatment of lung disease.

As used herein, the term "lung disease" refers to any disease in which abnormalities occur in tissues or functions of the lung, and specifically, the triple agonist of the present invention or a long-acting conjugate thereof can inhibit inflammation and fibrotic reactions, and thus, for the purpose of the present invention, the lung disease may include a disease accompanied by lung inflammation and fibrosis or a disease which is affected by fibrosis, for example, interstitial lung disease (ILD), progressive fibrosing interstitial lung disease (PF-ILD), idiopathic interstitial pneumonias (IIP), non-specific interstitial pneumonia (NSIP), pulmonary fibrosis, fibrosing interstitial lung diseases (FILD, idiopathic pulmonary fibrosis (IPF), alveolitis, pneumonia, emphysema, bronchitis, chronic obstructive pulmonary disease, combined pulmonary fibrosis and emphysema (CPFE), asthma, or respiratory infectious disease, *etc.,* but is not limited thereto.

The triple agonist of the present invention inhibits the activity of macrophages, the major mechanism of lung inflammation, and inhibits the differentiation of fibroblasts into myofibroblasts and the epithelial mesenchymal transition of alveolar epithelial cells, which are the major mechanisms of pulmonary fibrosis, thereby showing a preventive or therapeutic effect on lung disease, but is not limited thereto.

Specifically, the pharmaceutical composition of the present invention, when administered, may (i) inhibit the activity of macrophages; and/or (ii) reduce the expression of IL-1β, IL-6, IL-12, or TNF-α, thereby inhibiting inflammation, but is not limited thereto. In addition, although not limited thereto, the pharmaceutical composition of the present invention may have one or more of the following features upon administration:
(i) inhibits myofibroblast differentiation;
(ii) reduces the expression of α-SMA, collagen 1α1, or fibronectin;
(iii) inhibits epithelial mesenchymal transition (EMT) of alveolar epithelial cells; and
(iv) reduces the expression of collagen 1α1 or collagen 1α3. Through this, the pharmaceutical composition may inhibit fibrosis, but is not limited thereto.

The macrophages of the alveoli are involved during the inflammatory response in the lungs, and neutrophils are attracted by inflammatory cytokines (IL-1, IL-6, TNF-α) secreted by the macrophages, and proteases are secreted. In particular, elastase that decomposes elastin, a type of fiber that constitutes lung tissue and is involved in elasticity, is secreted. It is known that the elastin, which is an elastic tissue of the alveoli, is damaged by the elastase, and eventually the alveoli are also damaged, leading to fibrosis of the tissue. Although inflammation and fibrosis in the lungs become lung diseases themselves, the progression and deepening of inflammation and fibrosis can lead to other lung diseases, and therefore, inhibition of inflammation and fibrosis is required in the prevention and treatment of lung diseases.

The features of the triple agonist of the present invention mean the effects of inhibiting and improving inflammation and fibrosis of the lung, which also suggests a preventive or therapeutic effect on lung diseases accompanied by inflammation or fibrosis of the lung.

As used herein, the term chronic obstructive pulmonary disease (COPD) refers to a disease that shows irreversible airway obstruction, and is known to cause destruction of the lung parenchyma and pulmonary fibrosis due to chronic inflammation and damage. Infiltration of various inflammatory cells is observed in the airways of patients with chronic obstructive pulmonary disease, and in particular, it is known that the number of macrophages increases according to the severity of the disease, suggesting that inflammation plays an important role in the pathogenesis of chronic obstructive pulmonary disease. When harmful substances are inhaled, innate immunity is activated, and epithelial cells secrete many inflammatory mediators, which act to activate alveolar macrophages and neutrophils, thus, the control of the inflammatory response is an important factor in the treatment of chronic obstructive pulmonary disease. In general, the chronic obstructive pulmonary disease is divided into two types, i.e., emphysema and chronic bronchitis, but it is known that emphysema and chronic bronchitis coexist in many cases in actual patients.

As used herein, the term chronic bronchitis refers to a respiratory disease in which chronic inflammation occurs in the bronchial tubes, leading to enlargement and increase of mucous glands in the airways, or structural damage due to inflammation. As a result, the mucus layer thickens and ciliary movement is reduced, which blocks the flow of air, thereby causing breathing difficulties, *etc.*

As used herein, the term emphysema refers to a disease in which the bronchial tubes or lungs become inflamed due to various causes, which results in the secretion of proteases, destruction of the basic skeleton of the alveoli, damage to the vascular structure, and loss of gas exchange function.

As used herein, the term alveolitis refers to a disease in which the alveoli, which are responsible for oxygen exchange, become inflamed. When macrophages accumulate in the alveoli due to mold, dust, petroleum, toluene, acetone, chemicals, *etc.,* and the alveolar septum thickens, it can lead to alveolitis. As the alveolitis progresses, the alveoli are destroyed and become hardened due to scars, causing breathing difficulties.

As used herein, the term asthma, unlike chronic obstructive pulmonary disease, is an inflammatory airway obstructive disease that causes breathing difficulties induced by airway inflammation, while exhibiting a reversible lesion. It is known that the airway of asthma patients secretes lots of cytokines such as IL-4, IL-5, IL-13, *etc.,* and the proliferation and hypertrophy of bronchial smooth muscles are significantly observed. As with chronic obstructive pulmonary disease, the control of the inflammatory response is important in treatment.

As used herein, the term pneumonia refers to a disease in which the parenchymal tissue of the lung or the alveoli are inflamed. It is caused by bacterial infection, viruses, protozoa, fungi, chemicals, *etc.,* and is a high-risk disease that can accompany various complications. It is known that pneumonia occurs when the immune mechanism (typically, alveolar macrophages) of the respiratory organs against pathogens does not function properly, or the level of the pathogens exceeds the limit that can be protected by the normal immune mechanism. It is also known that patients with chronic lung diseases such as asthma, chronic obstructive pulmonary disease, emphysema, bronchiectasis, *etc.,* are more likely to develop pneumonia. In the present invention, pulmonary inflammation or pneumonia may include pulmonary inflammation or pneumonia caused by or accompanied by other lung diseases.

As used herein, the term respiratory infectious disease refers to a respiratory disease caused by infection of pathogens (viruses, bacteria, fungi, *etc.).* The respiratory infectious disease may be classified according to pathogens that cause the disease. Representative causes of the respiratory infections include respiratory viruses, bacteria, mycoplasma, fungi, *etc.* Since the respiratory infectious disease caused by infection of pathogens is accompanied by inflammation, a therapeutic effect through improvement of inflammation can be expected.

As used herein, the term respiratory viral infectious disease refers to a respiratory disease caused by pathogenic viral infections, and it can cause severe lower respiratory tract infections with pneumonia and bronchitis from the mild upper respiratory tract infections. Respiratory viral infections are known to be fatal in patients with reduced cardiorespiratory function. When infected with a respiratory virus, the respiratory tract including the lungs is inflamed, and if the inflammation is not suppressed for a long period of time, it leads to fibrosis, resulting in a more serious lung disease. Therefore, it is important to treat respiratory viral infections while suppressing inflammation and fibrosis.

The respiratory viruses that cause respiratory viral infections may be adenovirus, vaccinia virus, herpes simplex virus, parainfluenza virus, rhinovirus, varicella zoster virus, measles virus, respiratory syncytial virus, Dengue virus, HIV (human immunodeficiency virus), influenza virus, coronavirus, severe acute respiratory syndrome associated virus (SARS-associated virus), or middle east respiratory syndrome coronavirus (MERS-CoV), but are not limited thereto.

One non-limiting example of the coronavirus may be SARS-CoV-2, and SARS-CoV-2 infection may cause coronavirus disease-2019 (COVID-19).

As used herein, the term coronavirus disease-2019 (COVID-19) refers to a viral infectious disease caused by coronavirus (2019-nCoV or SARS-CoV-2) infection. Although the clear source of infection and the route of infection have not been confirmed yet, it has led to a global pandemic due to rapid transmission. Coronavirus, which is a virus that can infect humans and various animals, is an RNA virus with a gene size of 27 kb to 32 kb, and is known to mainly show respiratory symptoms such as cough with fever, breathing difficulties, shortness of breath, sputum, *etc.*

In particular, the reason that coronavirus disease-2019 accompanies severe pneumonia is that the coronavirus attacks bronchial ciliary epithelial cells or Type II alveolar epithelial cells (type 2 epithelial cells in the alveoli). These cells have a large amount of enzyme receptors that make the coronavirus stick well. The receptors such as "ACE2", "TMPRSS2", *etc.* enhance the ability of coronavirus to penetrate cells.

Foreign substances or pathogens that have penetrated during respiration adhere to the mucous membrane of the ciliated epithelial cells of the bronchi. The ciliated epithelial cells, as the name suggest, have a large number of cilia, which expel pathogens including coronavirus adhered to the mucous membrane in the direction of the mouth and nose. However, there is a limit to the expulsion of a large number of viruses at once only by ciliary movement of the ciliated epithelial cells. In addition, smoking, dust, dry weather, low temperatures, *etc.* reduce ciliary movement, and also, the ciliary movement is decreased in a dry and cold winter, increasing influenza viral infections, including coronavirus disease-19.

Like other viruses, the coronavirus seizes the resource and system of a host cell, proliferates vigorously, and is ejected out of the infected cell. At this time, the exponentially proliferated viruses escape and rapidly infiltrate into surrounding healthy ciliated epithelial cells and Type II alveolar epithelial cells. Infected cells secrete cytokines that cause strong inflammation and turn into inflammatory cells.

The original function of Type II alveolar epithelial cells is to secrete surfactants to keep the alveoli taut and at the same time, to facilitate gas exchange through Type I alveolar epithelial cells. Type II alveolar epithelial cells attacked by the coronavirus change into inflammatory cells and lose their main function, causing inflammation (pneumonia) in the lungs, and accordingly, secondary symptoms (fever, cough, breathing difficulties, *etc.)* occurs.

The triple agonist of the present invention can inhibit the inflammatory response by inhibiting the activity of macrophages and/or reducing the expression level of inflammatory cytokines (IL-1β, IL-6, IL-12, or TNF-α) in lung tissue. Therefore, it can exhibit a preventive or therapeutic effect on lung diseases caused by or accompanied by inflammation (e.g., alveolitis, pneumonia, emphysema, bronchitis, chronic obstructive pulmonary disease, asthma, or respiratory infection disease).

Meanwhile, fibrosis is a disease that forms excessive fibrous connective tissues in an organ or tissue. Fibrosis refers to a state in which normal control is impossible during the wound healing process after the tissues in the human body are damaged by an inflammatory reaction caused by various factors (infection, chemical stimulation, radiation, *etc.).* Particularly in the lungs, when inflammation of the lungs occurs and persists for a long time without being treated, the tissue is not regenerated, which leads to thickened fibrosis, resulting in serious lung disease in many cases. Accordingly, since it is necessary to suppress inflammation in the treatment of fibrosis to prevent the progression of fibrosis, immunosuppressants (e.g., steroids, cytoxan), *etc.,* are used as therapeutic agents. Herein, fibrosis may be used interchangeably with fibrosis.

As used herein, the term pulmonary fibrosis refers to a condition in which the proliferation of fibrous connective tissue in the lungs occurs, causing destruction of the normal lung structure, and hardening and devastation of the lung tissues. There are parenchymal, interstitial and mixed types, but interstitial pulmonary fibrosis is particularly problematic, in which proliferation of fibrous connective tissue appears around the alveolar wall and bronchioles. In general, it is known that transforming growth factor-β (TGF-β) is produced in various cells such as alveolar macrophages, activated alveolar epithelial cells, fibroblasts, and myofibroblasts, fibroblast proliferation and migration of macrophages and fibroblasts are induced, and the expression of inflammatory and fibrotic cytokines such as TNF-α, PDGF, IL-1β, and IL-13 is stimulated to further enhance the fibrosis response (Proc Am Thorac Soc., 9(3):111-116 (2012)). The pulmonary fibrosis of the present invention may include, but is not limited to, pulmonary fibrosis caused by or accompanied by other lung diseases.

As used herein, the term combined pulmonary fibrosis and emphysema (CPFE) is a representative disease in which fibrosis coexists with emphysema.

As used herein, the term interstitial lung disease (ILD) is also known as diffuse parenchymal lung disease (DPLD), and is a generic term for diseases that show abnormal collagen deposition accompanied by proliferation of the lung interstitial compartment, infiltration of inflammatory cells, and fibrosis. The interstitial lung disease is classified into occupational, environmental, anthropogenic, connective tissue, or idiopathic disease according to the cause thereof.

As used herein, the term progressive fibrosing interstitial lung disease (PF-ILD) refers to chronic fibrotic interstitial lung disease showing a progressive phenotype, and may include autoimmune interstitial lung disease, systemic sclerosis-associated interstitial lung disease, mixed connective tissue disease-associated interstitial lung disease, non-specific idiopathic interstitial pneumonia, unclassified idiopathic interstitial pneumonia, *etc.,* but is not limited thereto.

Among the interstitial lung diseases, idiopathic interstitial pneumonia (IIP), for which the causes are unknown, refers to lung diseases classified according to the histological types that invade the lung interstitium, and representative examples thereof include non-specific interstitial pneumonia (NSIP) and idiopathic pulmonary fibrosis (IPF).

As used herein, the term idiopathic pulmonary fibrosis (IPF) is the most common disease among idiopathic interstitial pneumonia, and is defined as pulmonary fibrosis for which the exact cause is unknown. It is known that after the alveolar epithelial cells are damaged by repeated inflammatory reactions due to various exposures, fibrosis is induced as the wounds are not normally treated, and pulmonary fibrosis progresses by a complex interaction between genetic predisposition, environmental factors, and lung infection. Specifically, the proliferation of fibroblasts/myofibroblasts in the lungs by various factors secreted from damaged alveolar epithelial cells and infiltrated inflammatory cells, secretion and accumulation of collagen caused thereby, and excessive deposition of extracellular matrix (ECM) are the pathogenetic mechanisms of the disease.

Since the triple agonist of the present invention exhibits one or more features of (i) inhibiting myofibroblast differentiation; (ii) reducing the expression of α-SMA, collagen 1α1, or fibronectin; (iii) inhibiting epithelial mesenchymal transition (EMT) of alveolar epithelial cells; and (iv) reducing the expression of collagen 1α1 or collagen 1α3, it can inhibit and improve fibrosis, and may exhibit a preventive or therapeutic effect on lung diseases caused by or accompanied by fibrosis (e.g., interstitial lung disease, progressive fibrosing interstitial lung disease, idiopathic interstitial pneumonia, non-specific interstitial pneumonia, pulmonary fibrosis, interstitial pulmonary fibrosis, idiopathic pulmonary fibrosis, combined pulmonary fibrosis and emphysema).

Meanwhile, not only the triple agonist according to the present invention can exhibit a therapeutic effect on coronavirus disease-19, it is known that symptoms of pulmonary fibrosis remain as sequelae even after being cured of coronavirus disease-19, and the triple agonist of the present invention exhibits an effect of improving pulmonary fibrosis, and thus, the triple agonist may exhibit efficacy on pulmonary inflammation and/or pulmonary fibrosis caused by coronavirus disease-19.

The pharmaceutical composition of the present invention may be further administered with a mucolytic agent or a pharmaceutically acceptable salt thereof, but is not limited thereto. For the purposes of the present invention, the pharmaceutical composition is for co- administration of the triple agonist and mucolytic agent, and the triple agonist and mucolytic agent may be administered simultaneously, sequentially, or in reverse order, but is not limited thereto.

In the present invention, when the term "co-administration" is used, it should be understood that the components are administered simultaneously, individually, or sequentially. When administration is performed sequentially or individually, the interval of the administration of the second component should be set such that the beneficial effects of co-administration are not lost. The co-administration of the triple agonist and mucolytic agent may be performed as follows, but is not limited thereto:
a) (i) the triple agonist or a conjugate thereof and (ii) a mucolytic agent or a pharmaceutically acceptable salt thereof are administered as a mixture; or
b) (i) the triple agonist or a conjugate thereof and (ii) a mucolytic agent or a pharmaceutically acceptable salt thereof are administered in an isolated form.

When the triple agonist and the mucolytic agent or a pharmaceutically acceptable salt thereof are in an isolated form, the triple agonist and the mucolytic agent or a pharmaceutically acceptable salt thereof may be formulated as separate preparations and administered simultaneously, individually, sequentially, or in reverse order.

In the present invention, co-administration does not simply refer to concurrent administration, but may be understood as administration in a dosage form wherein the triple agonist and the mucolytic agent or a pharmaceutically acceptable salt thereof can act together on an individual such that each substance may perform functions at a level identical to or higher than natural functions thereof.

In a specific example, the triple agonist and the mucolytic agent or a pharmaceutically acceptable salt thereof may be mixed and co-administered as one formulation, alternatively, the triple agonist and the mucolytic agent or a pharmaceutically acceptable salt thereof may be individually formulated and co-administered simultaneously, sequentially, or in reverse order, but administration is not limited thereto. When formulated individually and co-administered, each formulation may be administered by different routes, but is not limited thereto. In addition, although not limited thereto, the triple agonist and the mucolytic agent or a pharmaceutically acceptable salt thereof may be individually formulated and included in a single kit. The pharmaceutically acceptable salt of the mucolytic agent may include a salt derived from pharmaceutically acceptable inorganic acids, organic acids, or bases. Examples of the suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* The salts derived from suitable bases may include alkali metals (e.g., sodium, potassium, *etc.*); alkali earth metals (e.g., magnesium); ammonium, *etc.*

As used herein, the term "mucolytic agent" refers to a drug that promotes the secretion, liquefaction, or expulsion of sputum of phlegm or mucus from the respiratory tract, in particular, a drug that acts to break down mucus in the lungs to only dilute respiratory secretions. In the present invention, the mucolytic agent may be used interchangeably with an expectorant.

Specific examples of the mucolytic agent of the present invention may include any one or more selected from the group consisting of ambroxol, *N*-acetylcysteine, *N-*acetylin, carbocysteine, domiodol, fudosteine, bromhexine, erdosteine, letostine, lysozyme, mesna, sobrerol, stepronin, tiopronin, tyloxapol, carbocisteine, dornase alfa, eprazinone, letosteine, neltenexine, and mecysteine, but the mucolytic agent is not limited thereto.

Meanwhile, the co-administration of the triple agonist and the mucolytic agent may exhibit a therapeutic effect on respiratory infectious diseases (e.g., coronavirus disease-19). Since the triple agonist of the present invention exhibits an effect of improving lung inflammation and pulmonary fibrosis, the co-administration of the triple agonist and the mucolytic agent may show efficacy on respiratory infectious diseases (e.g., coronavirus disease-19 (COVID-19)), or pulmonary inflammation and pulmonary fibrosis caused thereby.

The pharmaceutical composition of the present invention may further contain a pharmaceutically acceptable carrier or diluent. The pharmaceutically acceptable carrier or diluent may be one that is non-naturally occurring.

As used herein, the term "pharmaceutically acceptable" refers to the properties of having a sufficient amount to show a therapeutic effect and not causing adverse effects, and may be easily determined by those skilled in the art based on the factors well known in the medical field, such as the kind of disease, age, body weight, health status, sex, drug sensitivity of a patient, administration route, administration method, administration frequency, duration of treatment, a drug(s) to be mixed or administered simultaneously, *etc.*

The pharmaceutical composition of the present invention containing the peptide may further contain a pharmaceutically acceptable excipient. The pharmaceutically acceptable excipient may include, but is not particularly limited to, for oral administration, a binder, a lubricant, a disintegrant, a solubilizing agent, a dispersant, a stabilizing agent, a suspending agent, a coloring agent, a fragrance, *etc.*; for injections, a buffering agent, a preservative, an analgesic, a solubilizing agent, an isotonic agent, a stabilizing agent, *etc.,* which may be used in combination; and for topical administrations, a base, an excipient, a lubricant, a preservative, *etc.*

The formulation type of the composition of the present invention may be prepared variously by being combined with a pharmaceutically acceptable excipient described above. For example, for oral administration, the composition may be formulated into tablets, troches, capsules, elixirs, suspensions, syrups, wafers, *etc.,* and for injections, the composition may be formulated into unit-dose ampoules or multi-dose forms. The composition may also be formulated into solutions, suspensions, tablets, pills, capsules, sustained-release formulations, *etc.*

Meanwhile, examples of suitable carriers, excipients, and diluents for formulations may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anti-coagulant, a lubricant, a humectant, a fragrance, a preservative, *etc.*

Additionally, the pharmaceutical composition of the present invention may have any one formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Additionally, the composition may be formulated into a preparation of a unit dosage form suitable for the administration into a patient's body, and may specifically be formulated into a preparation useful for protein drugs according to the conventional method in the pharmaceutical field so as to be administered by an oral or parenteral route including skin, intravenous, intramuscular, intraarterial, intramedullary, intrathecal, intraventricular, pulmonary, transdermal, subcutaneous, intraperitoneal, intranasal, intragastrical, topical, sublingual, vaginal, or rectal route using the administration method commonly used in the art, but the administration routes are not limited thereto.

Additionally, the conjugate may be used by being mixed with various pharmaceutically acceptable carriers such as physiological saline or organic solvents. To increase stability or absorptivity, carbohydrates (e.g., glucose, sucrose, or dextran), antioxidants (e.g., ascorbic acid or glutathione), chelating agents, low-molecular weight proteins, or other stabilizers, *etc.* may be used as pharmaceutical drugs.

In another aspect, the present invention provides a method for the prevention or treatment of lung disease, which includes administering a composition containing a peptide including an amino acid sequence of any one of SEQ ID NOS: 1 to 102 or a long-acting conjugate thereof in a pharmaceutically effective amount.

The administration dose and frequency of the pharmaceutical composition of the present invention are determined by the type of drugs (i.e., active ingredients), together with various related factors, such as the disease to be treated, administration route, patient's age, sex, and body weight, severity of the disease, *etc.* Specifically, the composition of the present invention may be one which contains the triple agonist or a long-acting conjugate containing the same in a pharmaceutically effective amount, but the composition is not limited thereto.

Containing the peptide or a long-acting conjugate thereof in a pharmaceutically effective amount refers to a level at which the desired pharmacological activity (e.g., prevention, improvement, or treatment of lung disease) can be obtained by the triple agonist or a long-acting conjugate thereof, and in addition, may refer to a pharmaceutically acceptable level, which is a level at which toxicities or adverse effects do not occur or occur at an insignificant level in the subject to be administered, but the level is not limited thereto. The pharmaceutically effective amount as such may be determined by comprehensively considering the number of administration, patient, formulations, *etc.*

Although not particularly limited, the pharmaceutical composition of the present invention may contain the components (active ingredients) in an amount of 0.01% to 99% by weight to volume.

The total effective amount of the composition of the present invention may be administered to a patient in a single dose or may be administered for a long period of time in multiple doses according to a fractionated treatment protocol. In the pharmaceutical composition of the present invention, the content of active ingredients may vary depending on the severity of the disease. Specifically, the total daily dose of the triple agonist of the present invention or a long-acting conjugate thereof may be about 0.0001 mg to 500 mg per 1 kg of the body weight of a patient. However, the effective dose of the triple agonist or a conjugate thereof is determined considering various factors including patient's age, body weight, health conditions, sex, disease severity, diet, and excretion rate, as well as administration route and treatment frequency of the pharmaceutical composition. In this respect, those skilled in the art may easily determine the effective dose suitable for a particular use of the composition of the present invention. The pharmaceutical composition according to the present invention is not particularly limited to the formulation type and administration route and mode, as long as it shows the effects of the present invention.

The pharmaceutical composition of the present invention has excellent *in vivo* duration of efficacy and titer, and thus, the number and frequency of administration of the pharmaceutical preparation of the present invention can be significantly reduced.

Another aspect for implementing the present invention provides a method for the prevention or treatment of lung disease, which includes administering the triple agonist(peptide) and/or a long-acting conjugate of the triple agonist, or a composition containing the same to a subject in need thereof.

The triple agonist and/or a long-acting conjugate of the triple agonist, or a composition containing the same, lung disease, prevention, and treatment are as described above.

In the present invention, the subject refers to a subject suspected of having a lung disease, and the subject suspected of having a lung disease refers to mammals including humans, rats, cattle, etc., which have or are at risk of developing the lung disease, but any subject which can be treated with the triple agonist and/or conjugate of the present invention or the composition containing the same is included without limitation. In particular, the subject may be a subject with inflammation and/or fibrosis of the lung, or may be a subject having a lung disease caused by or accompanied by inflammation and/or fibrosis, but is not limited thereto.

As used herein, the term "administration" refers to the introduction of a particular material into a subject by any appropriate method, and the administration route of the composition is not particularly limited, but may be any conventional route that enables delivery of the composition to the target in the body (e.g., intraperitoneal administration, intravenous administration, intramuscular administration, subcutaneous administration, intradermal administration, oral administration, topical administration, intranasal administration, intrapulmonary administration, intrarectal administration, *etc.).*

The method of the present invention may include administering a pharmaceutical composition containing the triple agonist or a long-acting conjugate thereof in a pharmaceutically effective amount. An appropriate total daily dose of the pharmaceutical composition may be determined within the scope of correct medical judgment by a practitioner, and the pharmaceutical composition may be administered once or several times in divided doses. However, for the purpose of the present invention, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, various factors including drugs used in combination or simultaneously with the specific compositions, and similar factors well known in the medical field.

Although not limited thereto, the pharmaceutical composition of the present invention may be administered once a week, once every 2 weeks, or once every 4 weeks.

Specific examples of the method for the prevention or treatment of lung disease of the present invention may include a method of co-administering the triple agonist (peptide) and/or a long-acting conjugate of the triple agonist, or a composition containing the same, and a mucolytic agent or a pharmaceutically acceptable salt thereof simultaneously, sequentially, or in reverse order, but the method is not limited thereto. The co-administration is the same as described above.

Still another aspect for implementing the present invention provides a use of a composition containing the triple agonist or a long-acting conjugate thereof in the preparation of a medicament for the prevention or treatment of lung disease.

The triple agonist and/or a long-acting conjugate of the triple agonist, or a composition containing the same, lung disease, prevention, and treatment are as described above.

Yet another aspect for implementing the present invention provides a use of the triple agonist or a long-acting conjugate thereof, or a composition containing the same for the prevention or treatment of lung disease.

The triple agonist and/or a long-acting conjugate of the triple agonist, or a composition containing the same, lung disease, prevention, and treatment are as described above.

The use for the prevention or treatment of lung disease of the present invention may be a use for co-administration of the triple agonist (peptide) and/or a long-acting conjugate of the triple agonist, or a composition including the same, and a mucolytic agent or a pharmaceutically acceptable salt thereof, but the use is not limited thereto. The co-administration is as described above.

Hereinafter, the present invention will be described in more detail with reference to the following Examples. However, these Examples are for illustrative purposes only and the scope of the invention is not limited by these Examples.

### Example 1: Preparation of Triple Agonists

Triple agonists showing activities to all of GLP-1, GIP, and glucagon receptors were prepared, and their amino acid sequences are shown in Table 1 below.

**[Table 1]**

| **SEQ ID NO:** | **Sequence** | **Informatio n** |
|---|---|---|
| 1 | HXQGTFTSDVSSYLDGQAAKEFIAWLVKGC | |
| 2 | HXQGTFTSDVSSYLDGQAQKEFIAWLVKGC | |
| 3 | H X Q G T F T S D V S S Y L L G Q A A K Q F I A W L V K G G G P S S G A P P P S C | |
| 4 | HXQGTFTSDVSSYLLGQQQKEFIAWLVKGC | |
| 5 | H X Q G T F T S D V S S Y L L G Q Q Q K E F I A W L V K G G G P S S G A P P P S C | |
| 6 | HXQGTFTSDVSSYLDGQAAKEFVAWLLKGC | |
| 7 | HXQGTFTSDVSKYLDGQAAKEFVAWLLKGC | |
| 8 | HXQGTFTSDVSKYLDGQAAQEFVAWLLKGC | |
| 9 | HXQGTFTSDVSKYLDGQAAQEFVAWLLAGC | |
| 10 | H X Q G T F T S D V S K Y L D G Q A A Q E F V A W L L A G G G P S S G A P P P S C | |
| 11 | CA G E G T F T S D L S K Y L D S R R Q Q L F V Q W L K A G G P S S G A P P P S H G | |
| 12 | CA G E G T F I S D L S K Y M D E Q A V Q L F V E W L M A G G P S S G A P P P S H G | |
| 13 | CA G E G T F I S D Y S I Q L D E I A V Q D F V E W L L A Q K P S S G A P P P S H G | |
| 14 | CAGQGTFTSDYSIQLDEIAVRDFVEWLKNGG PSSGAPPPSHG | |
| 15 | CA G Q G T F T S D L S K Q M D E E A V R L F I E W L K N G G P S S G A P P P S H G | |
| 16 | CA G Q G T F T S D L S K Q M D S E A Q Q L F I E W L K N G G P S S G A P P P S H G | |
| 17 | CA G Q G T F T S D L S K Q M D E E R A R E F I E W L L A Q K P S S G A P P P S H G | |
| 18 | CA G Q G T F T S D L S K Q M D S E R A R E F I E W L K N T G P S S G A P P P S H G | |
| 19 | CAGQGTFTSDLSIQYDSEHQRDFIEWLKDTG PSSGAPPPSHG | |
| 20 | CA G Q G T F T S D L S I Q Y E E E A Q Q D F V E W L K D T G P S S G A P P P S H G | |
| 21 | Y X Q G T F T S D Y S K Y L D E C R A K E F V Q W L L D H H P S S G Q P P P S | Ring formation |
| 22 | Y X Q G T F T S D Y S K C L D E K R A K E F V Q W L L D H H P S S G Q P P P S | Ring formation |
| 23 | Y X Q G T F T S D Y S K Y L D E C R A K E F V Q W L L A Q K G K K N D W K H N I T | Ring formation |
| 24 | Y X Q G T F T S D Y S K Y L D E C R A K E F V Q W L K N G G | Ring |
| | PSSGAPPPS | formation |
| 25 | HXQGTFTSDCSKYLDERAAQDFVQWLLDGG PSSGAPPPS | |
| 26 | H X Q G T F T S D C S K Y L D S R A A Q D F V Q W L L D G G P S S G A P P P S | |
| 27 | HXQGTFTSDYSKYLDERACQDFVQWLLDQG GPSSGAPPPS | |
| 28 | H X Q G T F T S D Y S K Y L D E K R A Q E F V C W L L A Q K G K K N D W K H N I T | |
| 29 | HXQGTFTSDYSKYLDEKAAKEFVQWLLNTC | Ring formation |
| 30 | HXQGTFTSDYSKYLDEKAQKEFVQWLLDTC | Ring formation |
| 31 | HXQGTFTSDYSKYLDEKACKEFVQWLLAQ | Ring formation |
| 32 | H X Q G T F T S D Y S K Y L D E K A C K D F V Q W L L D G G P S S G A P P P S | Ring formation |
| 33 | H X Q G T F T S D Y S I A M D E I H Q K D F V N W L L A Q K C | Ring formation |
| 34 | H X Q G T F T S D Y S K Y L D E K R Q K E F V N W L L A Q K C | Ring formation |
| 35 | H X Q G T F T S D Y S I A M D E I H Q K D F V N W L L N T K C | Ring formation |
| 36 | H X Q G T F T S D Y S K Y L C E K R Q K E F V Q W L L N G G P S S G A P P P S G | Ring formation |
| 37 | H X Q G T F T S D Y S K Y L D E C R Q K E F V Q W L L N G G P S S G A P P P S G | Ring formation |
| 38 | CA X Q G T F T S D K S S Y L D E R A A Q D F V Q W L L D G G P S S G A P P P S S | |
| 39 | HXQGTFTSDYSKYLDGQHAQCFVAWLLAGG GPSSGAPPPS | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | Ring formation |
| 43 | Y X Q G T F T S D Y S K Y L D E K R A K E F V Q W L L D H H C S S G Q P P P S | Ring formation |
| 44 | H G Q G T F T S D C S K Q L D G Q A A Q E F V A W L L A G G P S S G A P P P S | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | CAXQGTFTSDYSICMDEIHQKDFVNWLLNTK | Ring formation |
| 50 | H X Q G T F T S D Y S K Y L D E K R A K E F V Q W L L D H H P S S G Q P P P S C | Ring formation |
| 51 | HXQGTFTSDYSKYLDEKRQKEFVQWLLNTC | Ring formation |
| 52 | HXQGTFTSDYSKYLDEKRQKEFVQWLLDTC | Ring formation |
| 53 | HXEGTFTSDYSIAMDEIHQKDFVNWLLAQC | Ring |
| | | formation |
| 54 | HXEGTFTSDYSIAMDEIHQKDFVDWLLAEC | Ring formation |
| 55 | HXQGTFTSDYSIAMDEIHQKDFVNWLLAQC | Ring formation |
| 56 | HXQGTFTSDYSKYLDEKRQKEFVNWLLAQC | Ring formation |
| 57 | HXQGTFTSDYSIAMDEIHQKDFVNWLLNTC | Ring formation |
| 58 | H X Q G T F T S D Y S K Y L D E K R Q K E F V Q W L L N T K C | Ring formation |
| 59 | CA X Q G T F T S D Y S I C M D E K H Q K D F V N W L L N T K | Ring formation |
| 60 | CA X Q G T F T S D Y S I A M D E K H C K D F V N W L L N T K | Ring formation |
| 61 | CAXQGTFTSDYSIAMDEIACKDFVNWLLNTK | Ring formation |
| 62 | CA X Q G T F T S D K S K Y L D E R A A Q D F V Q W L L D G G P S S G A P P P S | |
| 63 | CAX Q G T F T S D C S K Y L D E R A A Q D F V Q W L L D G G P S S G A P P P S | |
| 64 | Y X Q G T F T S D Y S K Y L D E C A A K E F V Q W L L D H H P S S G Q P P P S | Ring formation |
| 65 | H X Q G T F T S D Y S K C L D E K R A K E F V Q W L L D H H P S S G Q P P P S | Ring formation |
| 66 | Y X Q G T F T S D Y S K Y L D E C R A K D F V Q W L L D H H P S S G Q P P P S | Ring formation |
| 67 | Y X Q G T F T S D Y S K Y L D E C A A K D F V Q W L L D H H P S S G Q P P P S | Ring formation |
| 68 | Y X Q G T F T S D Y S K C L D E K A A K E F V Q W L L D H H P S S G Q P P P S | Ring formation |
| 69 | Y X Q G T F T S D Y S K C L D E R A A K E F V Q W L L D H H P S S G Q P P P S | Ring formation |
| 70 | Y X Q G T F T S D Y S K C L D E K R A K D F V Q W L L D H H P S S G Q P P P S | Ring formation |
| 71 | Y X Q G T F T S D Y S K Y L D E R A C K D F V Q W L L D H H P S S G Q P P P S | Ring formation |
| 72 | Y X Q G T F T S D C S K Y L D E R A A K D F V Q W L L D H H P S S G Q P P P S | Ring formation |
| 73 | CA X Q G T F T S D Y S K Y L D E C R A K E F V Q W L L D H H P S S G Q P P P S | Ring formation |
| 74 | CA X Q G T F T S D Y S K C L D E K R A K E F V Q W L L D H H P S S G Q P P P S | Ring formation |
| 75 | Y X Q G T F T S D Y S K Y L D E K A A K E F V Q W L L D H H P S S G Q P P P S C | Ring formation |
| 76 | Y X Q G T F T S D Y S K Y L D E K R A K D F V Q W L L D H H P S S G Q P P P S C | Ring formation |
| 77 | Y X Q G T F T S D Y S K Y L D E K A A K D F V Q W L L D H H P S S G Q P P P S C | Ring formation |
| 78 | H X Q G T F T S D Y S K Y L D E K R Q K E F V Q W L L D T K C | Ring formation |
| 79 | H X E G T F T S D Y S I A M D E I H Q K D F V N W L L A Q K C | Ring formation |
| 80 | HXEGTFTSDYSIAMDEIHQKDFVDWLLAEKC | Ring formation |
| 81 | CA X Q G T F T S D Y S K Y L D E K R Q K E F V Q W L L N T C | Ring formation |
| 82 | CA X Q G T F T S D Y S K Y L D E K R Q K E F V Q W L L D T | Ring |
| | C | formation |
| 83 | CAXEGTFTSDYSIAMDEIHQKDFVNWLLAQC | Ring formation |
| 84 | CAXEGTFTSDYSIAMDEIHQKDFVDWLLAEC | Ring formation |
| 85 | CAXQGTFTSDYSIAMDEIHQKDFVNWLLAQC | Ring formation |
| 86 | CA X Q G T F T S D Y S K Y L D E K R Q K E F V N W L L A Q C | Ring formation |
| 87 | CAXQGTFTSDYSIAMDEIHQKDFVNWLLNTC | Ring formation |
| 88 | CA X Q G T F T S D Y S K Y L D E K R Q K E F V Q W L L N T K C | Ring formation |
| 89 | CA X Q G T F T S D Y S K Y L D E K R Q K E F V Q W L L D T K C | Ring formation |
| 90 | CA X E G T F T S D Y S I A M D E I H Q K D F V N W L L A Q K C | Ring formation |
| 91 | CA X E G T F T S D Y S I A M D E I H Q K D F V D W L L A E K C | Ring formation |
| 92 | CA X Q G T F T S D Y S I A M D E I H Q K D F V N W L L A Q K C | Ring formation |
| 93 | CA X Q G T F T S D Y S K Y L D E K R Q K E F V N W L L A Q K C . | Ring formation |
| 94 | CA X Q G T F T S D Y S I A M D E I H Q K D F V N W L L N T K C | Ring formation |
| 95 | Y X Q G T F T S D Y S K Y L D E K R A K E F V Q W L L C H H P S S G Q P P P S | Ring formation |
| 96 | Y X Q G T F T S D Y S K Y L D E K R A K E F V Q W L L D H C P S S G Q P P P S | Ring formation |
| 97 | Y X Q G T F T S D Y S K Y L D E K R A K E F V Q W L L D C H P S S G Q P P P S | Ring formation |
| 98 | Y X Q G T F T S D Y S K A L D E K A A K E F V N W L L D H H P S S G Q P P P S C | Ring formation |
| 99 | Y X Q G T F T S D Y S K A L D E K A A K D F V N W L L D H H P S S G Q P P P S C | Ring formation |
| 100 | Y X Q G T F T S D Y S K A L D E K A A K E F V Q W L L D Q H P S S G Q P P P S C | Ring formation |
| 101 | Y X Q G T F T S D Y S K A L D E K A A K E F V N W L L D Q H P S S G Q P P P S C | Ring formation |
| 102 | Y X Q G T F T S D Y S K A L D E K A A K D F V N W L L D Q H P S S G Q P P P S C | Ring formation |

In the sequences described in Table 1, the amino acid represented by X represents aminoisobutyric acid (Aib), which is a non-natural amino acid, and the underlined amino acids represent the formation of a ring between the underlined amino acids. Additionally, in Table 1, CA represents 4-imidazoacetyl, and Y represents tyrosine.

### Example 2: Preparation of Long-Acting Conjugate of Triple Agonists

For the pegylation of the cysteine residue of triple agonists (SEQ ID NOS: 21, 22, 42, 43, 50, 77, and 96) of Example 1 using PEG (10 kDa) having a maleimide group and an aldehyde group at both ends, i.e., maleimide-PEG-aldehyde (10 kDa, NOF, Japan), the triple agonists and the maleimide-PEG-aldehyde were reacted at a molar ratio of 1:1 to 3 at a protein concentration of 1 mg/mL to 5 mg/mL at low temperature for 0.5 to 3 hours. In particular, the reaction was performed in an environment in which 20% to 60% isopropanol was added to 50 mM Tris buffer (pH 7.5). Upon completion of the reaction, the reaction solutions were applied to SP sepharose HP (GE Healthcare, USA) to purify the triple agonists which were mono-pegylated on cysteine.

Then, the purified mono-pegylated triple agonists and an immunoglobulin Fc (homodimer of SEQ ID NO: 139) were reacted at a molar ratio of 1:1 to 5 at a protein concentration of 10 mg/mL to 50 mg/mL at 4°C to 8°C for 12 to 18 hours. The reaction was performed in an environment in which 10 mM to 50 mM sodium cyanoborohydride (a reducing agent) and 10% to 30% isopropanol were added to 100 mM potassium phosphate butter (pH 6.0). Upon completion of the reaction, the reaction solutions were applied to the Butyl sepharose FF purification column (GE Healthcare, USA) and Source ISO purification column (GE Healthcare, USA) to purify the conjugates including the triple agonists and the immunoglobulin Fc. The resulting purified long-acting conjugate has a structure in which the triple agonist peptide, a polyethylene glycol (PEG) linker, and the Fc dimer are covalently linked in a molar ratio of 1:1:1, and the PEG linker is linked to only one chain of the two polypeptide chains of the Fc dimer.

Meanwhile, in the immunoglobulin Fc, two monomers having the amino acid sequence of SEQ ID NO: 139 (consisting of 221 amino acids) form a homodimer through a disulfide bond between cysteine, the third amino acid of each monomer, and the monomers of the homodimer each independently form an internal disulfide bond between cysteines at positions 35 and 95 and an internal disulfide bond between cysteines at positions 141 and 199.

After the preparation, the purity analyzed by reverse phase chromatography, size exclusion chromatography, and ion exchange chromatography was shown to be 95% or higher.

In particular, the conjugate in which the triple agonist of SEQ ID NO: 21 and an immunoglobulin Fc were linked through the PEG linker was named as a "conjugate including the triple agonist of SEQ ID NO: 21 and an immunoglobulin Fc" or "long-acting conjugate of SEQ ID NO: 21", and these can be used interchangeably in the present invention.

In particular, the conjugate in which the triple agonist of SEQ ID NO: 22 and an immunoglobulin Fc were linked by the PEG linker was named as "the conjugate including the triple agonist of SEQ ID NO: 22 and an immunoglobulin Fc" or "long-acting conjugate of SEQ ID NO: 22", and these can be used interchangeably in the present invention.

In particular, the conjugate in which the triple agonist of SEQ ID NO: 42 and an immunoglobulin Fc were linked by the PEG linker was named as "the conjugate including the triple agonist of SEQ ID NO: 42 and an immunoglobulin Fc" or "long-acting conjugate of SEQ ID NO: 42", and these can be used interchangeably in the present invention.

In particular, the conjugate in which the triple agonist of SEQ ID NO: 43 and an immunoglobulin Fc were linked by the PEG linker was named as "the conjugate including the triple agonist of SEQ ID NO: 43 and an immunoglobulin Fc" or "long-acting conjugate of SEQ **ID** NO: 43", and these can be used interchangeably in the present invention.

In particular, the conjugate in which the triple agonist of SEQ **ID** NO: 50 and an immunoglobulin Fc were linked by the PEG linker was named as "the conjugate including the triple agonist of SEQ **ID** NO: 50 and an immunoglobulin Fc" or "long-acting conjugate of SEQ **ID** NO: 50", and these can be used interchangeably in the present invention.

In particular, the conjugate in which the triple agonist of SEQ **ID** NO: 77 and an immunoglobulin Fc were linked by the PEG linker was named as "the conjugate including the triple agonist of SEQ **ID** NO: 77 and an immunoglobulin Fc" or "long-acting conjugate of SEQ **ID** NO: 77", and these can be used interchangeably in the present invention.

In particular, the conjugate in which the triple agonist of SEQ **ID** NO: 96 and an immunoglobulin Fc were linked by the PEG linker was named as "the conjugate including the triple agonist of SEQ **ID** NO: 96 and an immunoglobulin Fc" or "long-acting conjugate of SEQ **ID** NO: 96", and these can be used interchangeably in the present invention.

### Experimental Example 1: Measurement of in vitro Activities of Triple Agonists and Long-Acting Conjugates Thereof

In order to measure the activities of the triple agonists and long-acting conjugates thereof prepared in Examples 1 and 2, a method of measuring *in vitro* cellular activities using cell lines where a GLP-1 receptor, a glucagon (GCG) receptor, and a GIP receptor are each transformed, was used.

Each of the cell lines above is one in which the genes for a human GLP-1 receptor, a human GCG receptor, and a human GIP receptor are transformed into Chinese hamster ovary (CHO), respectively, to be expressed therein, and is thus suitable for the measurement of the activities of GLP-1, GCG, and GIP. Accordingly, the activity for each part was measured using the respective transformed cell line.

For the measurement of the GLP-1 activities of the triple agonists and long-acting conjugates thereof prepared in Examples 1 and 2, human GLP-1 was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture solution was removed from the cultured CHO cells, in which the human GLP-1 receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers compared to human GLP-1 are shown in Tables 2 and 3 below.

For the measurement of the GCG activities of the triple agonists and long-acting conjugates thereof prepared in Examples 1 and 2, human GCG was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture solution was removed from the cultured CHO cells, in which the human GCG receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers compared to human GCG are shown in Tables 2 and 3 below.

For the measurement of the GIP activities of the triple agonists and long-acting conjugates thereof prepared in Examples 1 and 2, human GIP was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture solution was removed from the cultured CHO cells, in which the human GIP receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers compared to human GIP are shown in Tables 2 and 3 below.

**[Table 2]**

| Relative titer ratio of triple agonists | | | |
|---|---|---|---|
| | ***In vitro* Activity Compared to Native Peptide (%)** | | |
| **SEQ ID NO:** | **vs. GLP-1** | **vs. Glucagon** | ***vs.* GIP** |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | <0.1 | <0.1 |
| 14 | 28.0 | <0.1 | <0.1 |
| 15 | 79.2 | <0.1 | <0.1 |
| 16 | 2.1 | <0.1 | <0.1 |
| 17 | 0.2 | <0.1 | <0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | <0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | <0.1 | <0.1 |
| 46 | 1.4 | <0.1 | <0.1 |
| 47 | 2.4 | <0.1 | <0.1 |
| 48 | 1.5 | <0.1 | <0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**[Table 3]**

| Relative titer ratio of long-acting conjugates of triple agonists | | | |
|---|---|---|---|
| Long-Acting Conjugate | *In vitro* Activity Compared to Native Peptide (%) | | |
| | vs. GLP-1 | vs. Glucagon | vs. GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The novel long-acting conjugates of the triple agonists prepared above have the function of triple agonists which can activate all of GLP-1 receptors, GIP receptors, and glucagon receptors, and thus can be used as a therapeutic material for treating lung disease.

### Experimental Example 2: Confirmation of Inflammation-Improving Effect of Long-Acting Conjugate of Triple Agonist in Lung

If was confirmed whether the long-acting conjugates of the triple agonist according to the present invention prepared in the above Examples exhibits an effect of improving inflammation in the lung using mice administered with elastase (ELA).

Specifically, C57BL/6 mice (ORIENT Bio, Busan, Korea) treated with ELA at 0.2 U/mouse were divided into an excipient control group, a long-acting conjugate of SEQ **ID** NO: 42 administration group (hereinafter, the long-acting conjugate of triple agonist; 6.5 nmol/kg, Q2D, subcutaneous), and a roflumilast administration group, which is an anti-inflammatory agent and a therapeutic agent for COPD (10 mg/kg, QD, oral), and repeated administration was performed for 3 weeks. Thereafter, the degree of change in the expression of inflammatory cytokines in the lung tissue according to the administration of the excipient, the long-acting conjugate of the triple agonist, and roflumilast was confirmed through qPCR. Statistical treatment evaluated the efficacy of the long-acting conjugate of the triple agonist using one-way ANOVA (*-**p <0.05-0.01).

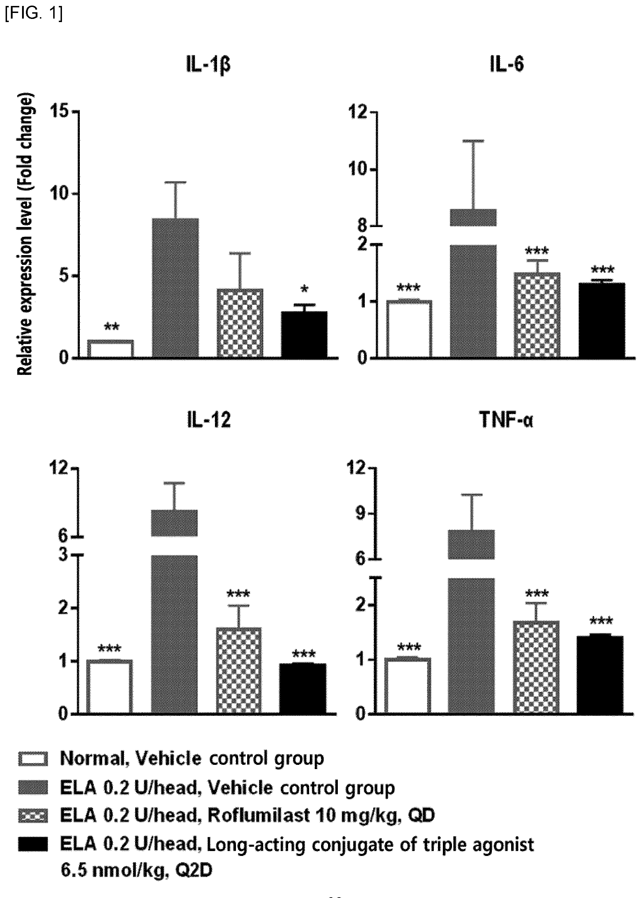

As a result, upon repeated administration of the long-acting conjugate of the triple agonist, the expression of IL-1β, IL-6, IL-12, and TNF-α in the lung tissue was reduced significantly and at a level equal to or greater than that of the excipient control group and the roflumilast administration group (FIG. 1).

Based on the results, it was confirmed that the long-acting conjugate of the triple agonist of the present invention has the effect of suppressing and improving inflammation in the lung.

### Experimental Example 3: Confirmation of Therapeutic Effect of Long-Acting Conjugate of Triple Agonist in Emphysema and Chronic Obstructive Pulmonary Disease

The present inventors have made attempts to confirm whether the long-acting conjugate of the triple agonist could show an *in vivo* therapeutic effect on emphysema and chronic obstructive pulmonary disease (COPD), which are typical lung diseases caused by lung inflammation.

To this end, an animal model, in which chronic obstructive pulmonary disease was induced by damage to lung tissue by intratracheal administration of elastase, was used. Specifically, C57BL/6 mice (ORIENT Bio, Busan, Korea) treated with elastase at 0.2 U/mouse were divided into an excipient control group, a long-acting conjugate of triple agonist administration group (6.5 nmol/kg, Q2D, subcutaneous), and a roflumilast administration group (10 mg/kg, QD, oral), and repeated administration was performed for 3 weeks. After repeated administration for 3 weeks, the lung tissue of each mouse was taken by autopsy, and the degree of emphysema of the lung tissue was evaluated through H&E staining.

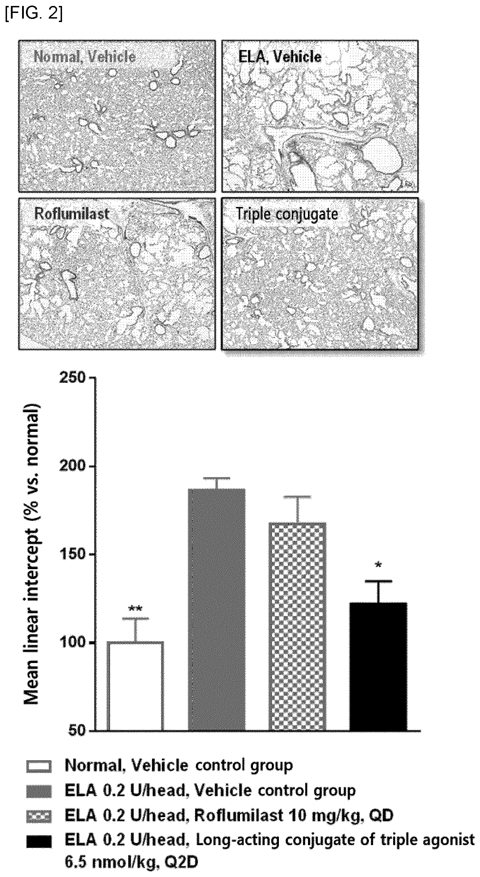

As a result, when the long-acting conjugate of the triple agonist was administered repeatedly, superior emphysema improvement efficacy was confirmed compared to the excipient control group and the roflumilast administration group, which is known as a therapeutic agent for COPD (*-**p < 0.05-0.01) (FIG. 2).

Based on the results, it was confirmed that the long-acting conjugate of the triple agonist of the present invention exhibits an effect of improving inflammation and emphysema in lung tissue, and has excellent therapeutic efficacy for chronic obstructive pulmonary disease.

### Experimental Example 4: Confirmation of Improvement Effect of Long-Acting Conjugate of Triple Agonist on Lung Fibrosis

In addition to the effect of improving inflammation in lung tissue confirmed in the above Examples, it was attempted to confirm whether the long-acting conjugate of the triple agonist according to the present invention also has an effect of improving lung fibrosis. Accordingly, MRC5 cell line, which is a lung fibroblast, and A549 cell line, which is an alveolar epithelial cell, were used to confirm the effect on the differentiation of lung fibroblasts into myofibroblasts and the epithelial mesenchymal transition (EMT) of alveolar epithelial cells, which are known to be important in the lung fibrosis process.

### Experimental Example 4-1: Confirmation of inhibitory effect on differentiation of lung fibroblasts into myofibroblast

First, MRC5 cells, which are lung fibroblasts, were divided into a vehicle-treated group and a long-acting conjugate of triple agonist-treated group, and TGF-β1 was co-treated to induce differentiation into myofibroblasts. As a negative control group, a group that was not treated with both of TGF-β1 and the long-acting conjugate of the triple agonist was used.

After 48 to 72 hours of co-treatment, the expression levels of α-SMA, collagen 1α1, and fibronectin, which are myofibroblast differentiation markers, in each group were confirmed through quantitative PCR. Statistical treatment evaluated the efficacy of the long-acting conjugate of the triple agonist using one-way ANOVA. (*-**p < 0.05-0.01).

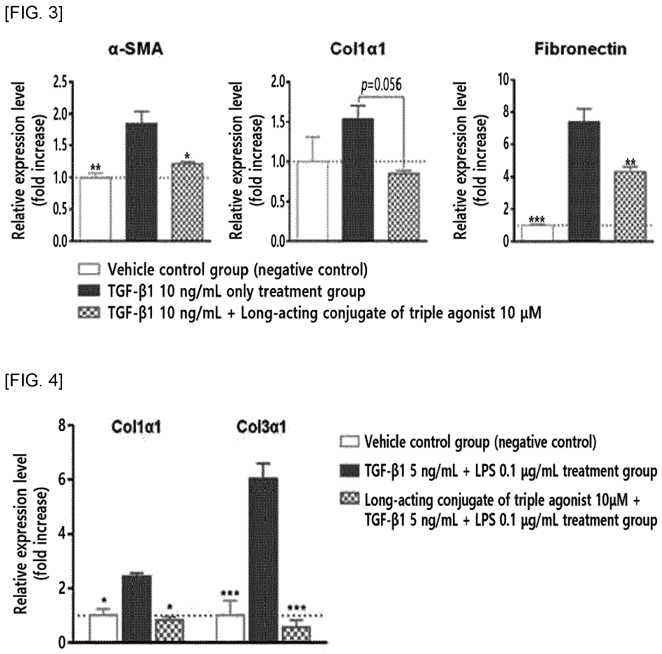

As a result, it was confirmed that when the long-acting conjugate of the triple agonist was co-treated with TGF-β1, the expression of the myofibroblast differentiation markers decreased compared to the excipient-treated group (FIG. 3).

### Experimental Example 4-2: Confirmation of the inhibitory effect on epithelial-mesenchymal transition (EMT) of alveolar epithelial cells

Then, the A549 cells were divided into an excipient-treated group and a long-acting conjugate of the triple agonist-treated group and subjected to pre-treatment, and subsequently treated with TGF-β1 and LPS sequentially to induce EMT. As a negative control group, a group that was not treated with all of the long-acting conjugate of the triple agonist, TGF-β1, and LPS was used.

After 48 hours, the expression levels of collagen 1α1 and collagen 3α1, which are EMT markers, in each group were confirmed through quantitative PCR. Statistical treatment evaluated the efficacy of the long-acting conjugate of the triple agonist using one-way ANOVA. (*-*** p < 0.05-0.001).

As a result, it was confirmed that the expressions of EMT markers in alveolar epithelial cells were significantly reduced when the long-acting conjugate of the triple agonist was pre-treated compared to the excipient-treated group (FIG. 4).

### Experimental Example 4-3: Confirmation of in vivo effect of improving fibrosis

In order to confirm the *in vivo* efficacy of the long-acting conjugate of the triple agonist prepared in the above Examples for improving pulmonary fibrosis, particularly idiopathic pulmonary fibrosis (IPF), which is a representative pulmonary fibrosis, bleomycin (BLM) mice were used. It is known that, when bleomycin is administered intratracheally, the epithelial-mesenchymal transition occurs by damaging DNA of alveolar epithelial cells, which induces idiopathic fibrosis. In addition, it is known that when the BLM treatment dose is increased above a certain level, the survival rate of the mice is reduced due to severe lung damage caused by idiopathic fibrosis.

Accordingly, the effect of improving lung fibrosis of the long-acting conjugate of the triple agonist in mice treated with BLM was confirmed, and furthermore, it was confirmed whether the survival rate could be increased through the improvement of lung fibrosis.

IPF mice prepared by treating C57BL/6 mice (ORIENT Bio, Busan, Korea) with BLM at 1.5 U/head were divided into an excipient control group, a long-acting conjugate of triple agonist administration group (3.9 nmol/kg, Q2D, subcutaneous), a pirfenidone administration group (300 mg/kg, QD, oral), and an ambroxol administration group (45 mg/kg, BID, peritoneal), repeated administration was performed for 2 weeks. After 2 weeks of repeated administration, lung tissue of each mouse was taken by autopsy, and the degree of fibrosis in the lung tissue was evaluated by Masson's trichrome staining. Statistical treatment evaluated the efficacy of the long-acting conjugate of the triple agonist using one-way ANOVA (*-****p* < 0.05-0.001).

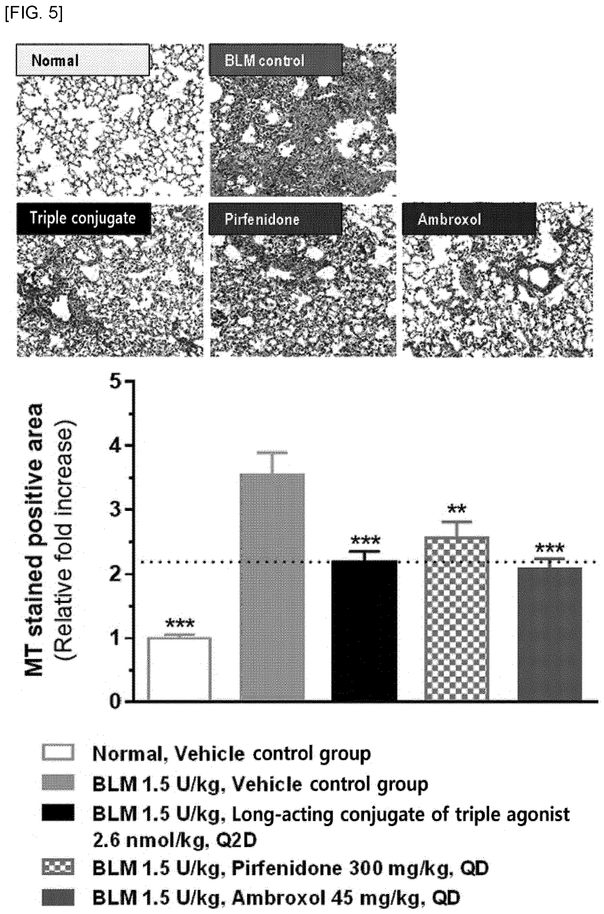

As a result, when the long-acting conjugate of the triple agonist was repeatedly administered, it was possible to confirm the superior efficacy in reducing the positive area of Masson's trichrome staining in the lung tissue as compared to the excipient control group and the pirfenidone administration group known as a therapeutic agent for IPF (*-***p < 0.05-0.001). In addition, in the case of ambroxol known as an antitussive expectorant, it was confirmed that it showed the same level of efficacy as the long-acting conjugate of the triple agonist (FIG. 5)

Further, IPF mice prepared by treating C57BL/6 mice (ORIENT Bio, Busan, Korea) with BLM at 3.0 U/head were divided into an excipient control group, a long-acting conjugate of triple agonist administration group (3.9 nmol/kg, Q2D, subcutaneous), a pirfenidone administration group (300 mg/kg, QD, oral), and an ambroxol administration group (45 mg/kg, BID, peritoneal), repeated administration was performed for 2 weeks. The survival rate during repeated administration was confirmed.

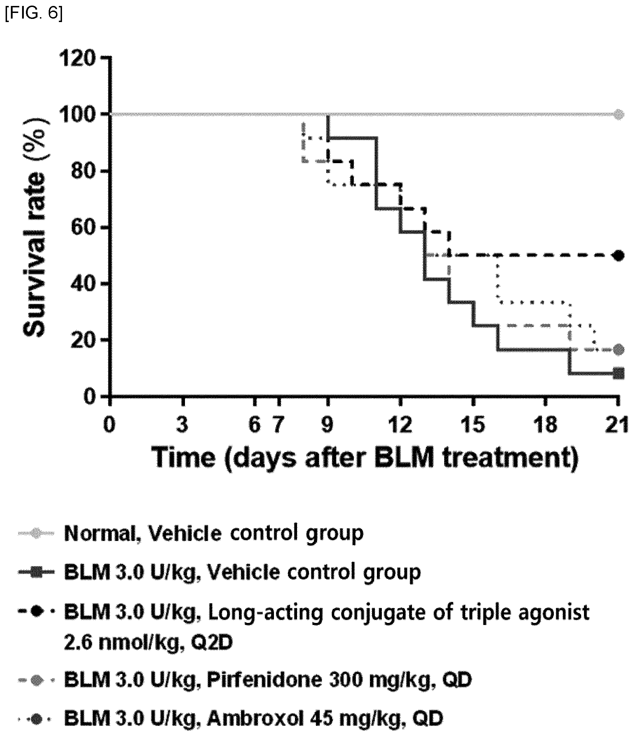

As a result, it was confirmed that, when the long-acting conjugate of the triple agonist was repeatedly administered, it showed superior survival rates compared to the excipient control group, the pirfenidone administration group, and the ambroxol administration group (FIG. 6).

Based on the results, it was confirmed that the long-acting conjugate of the triple agonist had a therapeutic effect on pulmonary fibrosis or idiopathic pulmonary fibrosis (IPF), thereby exhibiting efficacy in improving the survival rate.

It was confirmed that the long-acting conjugate of the triple agonist according to the present invention can improve lung fibrosis by inhibiting myofibroblast differentiation of lung fibroblasts and EMT of alveolar epithelial cells, and it was also confirmed that it can show efficacy in preventing and treating pulmonary fibrosis, particularly idiopathic pulmonary fibrosis, through the effect of improving pulmonary fibrosis *in vivo.*

In summary, the above results suggest that the conjugates of the triple agonists of the present invention can effectively prevent or treat related lung diseases through improvement of lung inflammation and pulmonary fibrosis, which implies that that they can be provided as new therapeutic agents for lung diseases.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A pharmaceutical composition for the prevention or treatment of lung disease, comprising:
a pharmaceutically acceptable excipient; and
a peptide comprising an amino acid sequence of any one of SEQ ID NOS: 1 to 102 in a pharmaceutically effective amount.

2. The pharmaceutical composition of claim 1, wherein the peptide is in the form of a long-acting conjugate, and the long-acting conjugate is represented by Formula 1 below:
[Formula 1] X-L-F
wherein in Formula 1 above,
X is a peptide of an amino acid sequence of any one of SEQ ID NOS: 1 to 102;
L is a linker comprising an ethylene glycol repeat unit;
F is an immunoglobulin Fc region; and
"-" represents a covalent bond between X and L and between L and F.

3. The pharmaceutical composition of claim 1 or 2, wherein the C-terminus of the peptide is amidated.

4. The pharmaceutical composition of claim 1 or 2, wherein the peptide comprises an amino acid sequence selected from the group consisting of SEQ ID NOS: 21, 22, 42, 43, 50, 64, 66, 67, 70, 71, 76, 77, 96, 97, and 100.

5. The pharmaceutical composition of claim 4, wherein the peptide comprises an amino acid sequence selected from the group consisting of SEQ ID NOS: 21, 22, 42, 43, 50, 77, and 96.

6. The pharmaceutical composition of claim 1 or 2, wherein the amino acids at positions 16 and 20 from the N-terminus form a ring with each other in the peptide sequence.

7. The pharmaceutical composition of claim 2, wherein the formula weight of the ethylene glycol repeat unit portion in the L is in the range of 1 kDa to 100 kDa.

8. The pharmaceutical composition of claim 2, wherein the F is an IgG Fc region.

9. The pharmaceutical composition of claim 1 or 2, wherein the lung disease is interstitial lung disease (ILD), progressive fibrosing interstitial lung disease (PF-ILD), idiopathic interstitial pneumonias (IIP), non-specific interstitial pneumonia (NSIP), pulmonary fibrosis, fibrosing interstitial lung diseases (FILD), idiopathic pulmonary fibrosis (IPF), alveolitis, pneumonia, emphysema, bronchitis, chronic obstructive pulmonary disease, combined pulmonary fibrosis and emphysema (CPFE), asthma, or respiratory infectious disease.

10. The pharmaceutical composition of claim 9, wherein the respiratory infectious disease is an infectious disease caused by respiratory viruses, bacteria, mycoplasma, or fungi.

11. The pharmaceutical composition of claim 10, wherein the respiratory virus is any one selected from the group consisting of adenovirus, vaccinia virus, herpes simplex virus, parainfluenza virus, rhinovirus, varicella zoster virus, measles virus, respiratory syncytial virus, Dengue virus, HIV (human immunodeficiency virus), influenza virus, coronavirus, severe acute respiratory syndrome associated virus (SARS-associated virus), and middle east respiratory syndrome coronavirus (MERS-CoV).

12. The pharmaceutical composition of claim 11, wherein the coronavirus is SARS-CoV-2.

13. The pharmaceutical composition of claim 1 or 2, wherein the pharmaceutical composition (i) inhibits the activity of macrophages; or (ii) reduces the expression of IL-1β, IL-6, IL-12, or TNF-α when administered.

14. The pharmaceutical composition of claim 1 or 2, wherein the pharmaceutical composition has one or more features when administered:
(i) inhibits myofibroblast differentiation;
(ii) reduces the expression of α-SMA, collagen 1α1, or fibronectin;
(iii) inhibits epithelial mesenchymal transition (EMT) of alveolar epithelial cells; and
(iv) reduces the expression of collagen 1α1 or collagen 1α3.

15. The pharmaceutical composition of claim 1 or 2, wherein the pharmaceutical is further administered with a mucolytic agent or a pharmaceutically acceptable salt thereof.

16. The pharmaceutical composition of claim 15, wherein the mucolytic agent is one or more selected from the group consisting of ambroxol, *N*-acetylcysteine, N-acetylin, carbocysteine, domiodol, fudosteine, bromhexine, erdosteine, letostine, lysozyme, mesna, sobrerol, stepronin, tiopronin, tyloxapol, carbocisteine, dornase alfa, eprazinone, letosteine, neltenexine, and mecysteine.

17. The pharmaceutical composition of claim 14, wherein the peptide and mucolytic agent or a pharmaceutically acceptable salt thereof are administered simultaneously, sequentially, or in reverse order.

18. The pharmaceutical composition of claim 9, wherein the lung disease is pneumonia (pulmonary inflammation) or pulmonary fibrosis caused by coronavirus disease-19 (COVID-19).

19. The pharmaceutical composition of claim 2, wherein the F is a dimer consisting of two polypeptide chains, and one end of L is linked to only one of the two polypeptide chains.
